(19) Europäisches Patentamt / European Patent Office / Office européen des brevets

(11) **EP 3 995 019 A1**

(12) **EUROPEAN PATENT APPLICATION**
published in accordance with Art. 153(4) EPC

(43) Date of publication:
**11.05.2022 Bulletin 2022/19**

(21) Application number: **20834275.8**

(22) Date of filing: **01.07.2020**

(51) International Patent Classification (IPC):
**A24F 47/00** (2020.01)    **A24F 40/50** (2020.01)

(52) Cooperative Patent Classification (CPC):
**A24F 40/50; A24F 47/00**

(86) International application number:
**PCT/JP2020/025857**

(87) International publication number:
**WO 2021/002392 (07.01.2021 Gazette 2021/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.07.2019 JP 2019124308**

(71) Applicant: **Japan Tobacco Inc.**
**Tokyo 105-6927 (JP)**

(72) Inventors:
• **YAMADA, Manabu**
  **Tokyo 130-8603 (JP)**
• **AKAO, Takeshi**
  **Tokyo 130-8603 (JP)**
• **ONO, Yasuhiro**
  **Tokyo 130-8603 (JP)**
• **TANAKA, Shujiro**
  **Tokyo 130-8603 (JP)**
• **KITAHARA, Minoru**
  **Tokyo 130-8603 (JP)**

(74) Representative: **Hoffmann Eitle**
**Patent- und Rechtsanwälte PartmbB**
**Arabellastraße 30**
**81925 München (DE)**

(54) **METHOD FOR OPERATING POWER SUPPLY UNIT FOR SUCTION DEVICE, POWER SUPPLY UNIT FOR SUCTION DEVICE, AND COMPUTER-READABLE MEDIUM**

(57)    A method for operating an electric power source unit 202 for an inhaler 100 is provided. the method comprises: making a sensor 212 detect a puff action performed by a user; measuring detected-time that is a puff action period during that the detected puff action is continued; correcting the measured detected-time, by using a time correction model that is based on a characteristic parameter associated with the puff action; calculating accumulated detected-time by accumulating the corrected detected-time; and estimating a remaining quantity level of an inhaled component source, based on the accumulated detected-time.

Fig. 1A

100A

EP 3 995 019 A1

**Description**

TECHNICAL FIELD

The present disclosure relates to a method and a program for operating an electric power source unit for an inhaler, and an electric power source unit for an inhaler. More specifically, it relates to a method and a program for operating an electric power source unit which is installed in an inhaler which is used for generating an inhaled component such as aerosol or flavor-added aerosol, and an electric power source unit for the inhaler.

BACKGROUND ART

In an inhaler such as an electronic cigarette, a nebulizer, or the like which is generally used, an inhaled component source is atomized by supplying electric power to a heater to thereby raise the temperature of the heater, in response to suction action of a user. In relation to control action such as that explained above, a method for grasping the consumed quantity (or the remaining quantity) of an inhaled component source, or judging exhaustion of the inhaled component source, by using obtained various kinds of data such as data of temperature, the quantity of supply of electric power, the electric resistance, and so on, has been known.

CITATION LIST

PATENT LITERATURE

PTL 1: Japanese Patent Application Public Disclosure No. 2014-501105
PTL 2: Japanese Patent Application Public Disclosure No. 2015-531600
PTL 3: Japanese Patent Application Public Disclosure No. 2017-538410
PTL 4: Japanese Patent Application Public Disclosure No. 2016-525367
PTL 5: Japanese Patent Application Public Disclosure No. 2019-500896
PTL 6: Japanese Patent Application Public Disclosure No. 2014-501107

SUMMARY OF INVENTION

TECHNICAL PROBLEM

One of objects of the present disclosure is to make it possible to grasp the level of the remaining quantity of an inhaled component source in an inhaler which is being used, by taking a characteristic of suction action of a user into consideration, for providing a user with a comfortable suction experience. Especially, one of the objects is to improve accuracy of judgment of depletion of the remaining quantity of the inhaled component source. It should be reminded that, in the following description, suction action performed by a user is referred to as "puff action" or, simply, "puff," and one of or each of an aerosol source and a flavor source is referred to as "an inhaled component source."

SOLUTION TO PROBLEM

For solving the above problems, in an embodiment, in a first aspect, a method for operating an electric power source unit for an inhaler is provided. The method comprises: making a sensor in the electric power source unit detect a puff action performed by a user; measuring detected-time that is time during that the detected puff action is continued; correcting the detected-time, based on a value of a characteristic parameter associated with the puff action; calculating accumulated detected-time by accumulating the corrected detected-time; and estimating remaining quantity levels (a remaining quantity level) of a flavor source and/or an aerosol source, based on lengths (a length) of the accumulated detected-time.

According to the above method, appropriate accumulated detected-time can be estimated, so that accuracy of estimation of the remaining quantity levels (level) of a flavor source and/or an aerosol source can be improved. Further, appropriate grasping and notifying of the remaining quantity can be realized.

A method in a second aspect comprises the method in the first aspect, and said correcting the detected-time is based on an atomization characteristic of an aerosol source in the inhaler. By using an atomization characteristic of an aerosol source, it becomes possible to construct an appropriate time correction model, and realize efficient estimation of the remaining quantity level.

A method in a third aspect comprises the method in the first or second aspect, and the characteristic parameter comprises detected-time. By using the detected-time to the characteristic parameter, it becomes possible to construct an appropriate time correction model, and realize efficient estimation of the remaining quantity level.

A method in a fourth aspect comprises the method in the third aspect, and said correcting comprises weighted calculation of the detected-time that uses a multiplier selected in relation to the detected-time. By applying weighted calculation of the detected-time, it becomes possible to construct an appropriate time correction model, realize efficient estimation of the remaining quantity level, and improve accuracy of estimation of the remaining quantity level.

A method in a fifth aspect comprises the method in the fourth aspect, and a predetermined first number is selected as the multiplier in the case that the detected-time is equal to or shorter than predetermined first time, and a second number, that is specified based on the first number, is selected as the multiplier in the case that the detected-time is longer than the first time, wherein the first number is smaller than the second number. By applying numbers such as the first number and the second number, it becomes possible to construct an appropriate time correction model, realize efficient estimation of the remaining quantity level, and improve accuracy of estimation of the remaining quantity level.

A method in a sixth aspect comprises the method in the fifth aspect, and the first time is one second. By the above construction, accuracy of estimation of the remaining quantity level can be improved.

A method in a seventh aspect comprises the method in any one of the first to sixth aspects, and further comprises measuring a puff action interval between two successive puff actions, and the characteristic parameter comprises the puff action interval. By using the puff action interval to the characteristic parameter, it becomes possible to construct an appropriate time correction model, and realize efficient estimation of the remaining quantity level.

A method in an eighth aspect comprises the method in the seventh aspect, and said correcting comprises addition of adjustment time, that is calculated based on the puff action interval and predetermined second time, to the detected-time. By applying the adjustment time to the detected-time, it becomes possible to construct an appropriate time correction model, realize efficient estimation of the remaining quantity level, and improve accuracy of estimation of the remaining quantity level.

A method in a ninth aspect comprises the method in the eighth aspect, and, in said correcting, the adjustment time is set to 0 when the puff action interval is longer than the second time. By setting the adjustment time to 0 and applying it to the detected-time, it becomes possible to construct an appropriate time correction model, realize efficient estimation of the remaining quantity level, and improve accuracy of estimation of the remaining quantity level.

A method in a tenth aspect comprises the method in the eighth or ninth aspect, and the second time is ten seconds. By the above construction, accuracy of estimation of the remaining quantity level can be improved.

A method in an eleventh aspect comprises the method in any one of the first to tenth aspects, and said estimating comprises judging that shortage in the remaining quantities (quantity) of the flavor source and/or the aerosol source has occurred, when the accumulated lengths (length) of detected-time have (has) reached predetermined threshold lengths of time (a predetermined threshold length of time). By applying judgment of occurrence of shortage in the remaining quantity, appropriate detection of the end of life can be realized.

A method in a twelfth aspect comprises the method in the eleventh aspect, and further comprises making a notifier, which is a component of the electric power source unit, operate to provide notification representing shortage in the remaining quantity, in response to the judgement of occurrence of shortage in the remaining quantities (quantity) of the flavor source and/or the aerosol source. By applying the step for providing notification representing shortage in the remaining quantity, appropriate notification of the end of life can be realized.

A program in a thirteen aspect makes an electric power source unit perform the method in any one of the first to twelfth aspects.

Further, in a fourteenth aspect, an electric power source unit, which comprises a sensor for detecting a puff action performed by a user and a controller, for an inhaler is provided. Regarding the electric power source unit, the controller performs: measurement of detected-time that is time during that a detected puff action is continued; correction of the detected-time, based on an atomization characteristic of an aerosol source in the puff action; calculation of accumulated detected-time by accumulating the corrected detected-time; and estimation of remaining quantity levels (a remaining quantity level) of a flavor source and/or the aerosol source, based on lengths (a length) of the accumulated detected-time.

According to the above electric power source unit, appropriate accumulated detected-time can be estimated, so that accuracy of estimation of remaining quantity levels (level) of the flavor source and/or the aerosol source can be improved. Further, appropriate grasping and notifying of the remaining quantity can be realized.

An electric power source unit in a fifteenth aspect comprises the electric power source unit in the fourteenth aspect, and the estimation of the remaining quantity level comprises judging that shortage in the remaining quantities (quantity) of the flavor source and/or the aerosol source has occurred, when the accumulated lengths (length) of detected-time have (has) reached predetermined threshold lengths of time (a predetermined threshold length of time). By applying judgment of occurrence of shortage in the remaining quantity, appropriate detection of the end of life can be realized.

By applying judgment of occurrence of shortage in the remaining quantity, appropriate detection of the end of life can be realized.

An electric power source unit in a sixteenth aspect comprises the electric power source unit in the fifteenth aspect, and further comprises a notifier, and the controller makes the notifier operate to provide notification representing shortage in the remaining quantity, in response to the judgement of occurrence of shortage in the remaining quantity. By applying the step for providing notification representing shortage in the remaining quantity, appropriate notification of the end of life can be realized.

An electric power source unit in a seventeenth aspect comprises the electric power source unit in any one of the fourteenth to sixteenth aspects, and a first atomization characteristic of the aerosol source is specified in advance based on relationship between a sample operation period of puff action and an atomization quantity. By applying the first atomization characteristic, it becomes possible to construct an appropriate time correction model, realize efficient estimation of the remaining quantity level, and improve accuracy of estimation of the remaining quantity level.

An electric power source unit in an eighteenth aspect comprises the electric power source unit in any one of the fourteenth to seventeenth aspects, and a second atomization characteristic of the aerosol source is specified based on relationship between a sample operation interval between two successive puff actions and an atomization quantity. By applying the second atomization characteristic, it becomes possible to construct an appropriate time correction model, realize efficient estimation of the remaining quantity level, and improve accuracy of estimation of the remaining quantity level.

Further, in a nineteenth aspect, a method for operating an electric power source unit for an inhaler is provided. The method comprises: making a sensor detect a puff action performed by a user; measuring detected-time that is a puff action period during that the detected puff action is continued; correcting the measured detected-time, by using a time correction model that is based on a characteristic parameter associated with the puff action; calculating accumulated detected-time by accumulating the corrected detected-time; and estimating a remaining quantity level of an inhaled component source, based on the accumulated detected-time.

According to the above method, by flexibly adjusting detected-time of puff action performed by a user, appropriate accumulated detected-time can be estimated and accuracy of estimation of the remaining quantity level of the inhaled component source can be improved. Further, appropriate grasping and notifying of the remaining quantity can be realized.

A method in a twentieth aspect comprises the method in the nineteenth aspect, and the time correction model is defined based on an atomization characteristic of the inhaled component source in the inhaler. By using an atomization characteristic of the inhaled component source, it becomes possible to construct an appropriate time correction model, and realize efficient estimation of the remaining quantity level.

A method in a twenty-first aspect comprises the method in the nineteenth or twentieth aspect, and the characteristic parameter comprises the puff action period. By applying the detected-time to the characteristic parameter, it becomes possible to construct an appropriate time correction model, and realize efficient estimation of the remaining quantity level.

A method in a twenty-second aspect comprises the method in the twenty-first aspect, and the time correction model based on the puff action period is defined to include maintaining the corrected detected-time to be the first time, when a value of the measured detected-time is first time. By the above construction, it becomes possible to construct a further appropriate time correction model, and further improve accuracy of estimation of the remaining quantity level of the inhaled component source.

A method in a twenty-third aspect comprises the method in the twenty-second aspect, and the time correction model based on the puff action period is defined to include reducing the measured detected-time in accordance with a first function of the detected-time, when the value of the measured detected-time is smaller than the first time. By the above construction, it becomes possible to construct a further appropriate time correction model, and further improve accuracy of estimation of the remaining quantity level of the inhaled component source.

A method in a twenty-fourth aspect comprises the method in the twenty-second or twenty-third aspect, and the first time is 2.4 seconds. By the above construction, it becomes possible to construct a further appropriate time correction model suitable to a device characteristic of the inhaler.

A method in a twenty-fourth aspect comprises the method in any one of the nineteenth to twenty-fourth aspects, and further comprises measuring a puff action interval between two successive puff actions, wherein the characteristic parameter comprises the puff action interval. By applying the puff action interval to the characteristic parameter, it becomes possible to construct a further appropriate time correction model, and realize efficient estimation of the remaining quantity level.

A method in a twenty-sixth aspect comprises the method in the twenty-fifth aspect, and the time correction model based on the puff action interval is defined to include adding adjustment time, that is calculated based on the puff action interval, to the detected-time. By the above construction, it becomes possible to construct a further appropriate time correction model, and further improve accuracy of estimation of the remaining quantity level of the inhaled component source.

A method in a twenty-seventh aspect comprises the method in the twenty-sixth aspect, and the time correction model based on the puff action interval is defined to include setting the adjustment time to 0, when a value of the puff action interval is larger than second time. By the above construction, it becomes possible to construct a further appropriate time correction model, and further improve accuracy of estimation of the remaining quantity level of the inhaled component source.

A method in a twenty-eighth aspect comprises the method in the twenty-seventh aspect, and the time correction model based on the puff action interval is defined to include calculating the adjustment time in accordance with a second function of the puff action interval, when the value of the puff action interval is equal to or smaller than the second time. By the above construction, it becomes possible to construct a further appropriate time correction model, and further improve accuracy of estimation of the remaining quantity level of the inhaled component source.

A method in a twenty-ninth aspect comprises the method in the twenty-seventh or twenty-eighth aspect, and the second time is 10 seconds. By the above construction, it becomes possible to construct a further appropriate time correction model suitable to a device characteristic of the inhaler.

A method in a thirtieth aspect comprises the method in any one of the nineteenth to twenty-ninth aspects, and said correcting the detected-time further updates, when a value of the detected-time corrected based on a value of the characteristic parameter is equal to or smaller than predetermined third time, the value of the corrected detected-time to the third time. By the above construction, it becomes possible to construct a further appropriate time correction model, and further improve accuracy of estimation of the remaining quantity level of the inhaled component source.

A method in a thirty-first aspect comprises the method in any one of the nineteenth to thirtieth aspects, and said estimating comprises judging that shortage in the remaining quantity of the inhaled component source has occurred, when the accumulated detected-time has reached predetermined fourth time. By applying judgment of occurrence of shortage in the remaining quantity, appropriate detection of the end of life can be realized.

A method in a thirty-second aspect comprises the method in the thirty-first aspect, and further comprises making a notifier, which is a component of the electric power source unit, operate to provide notification representing shortage in the remaining quantity, in response to the judgement of occurrence of shortage in the remaining quantity of the inhaled component source. By applying the construction for notifying shortage in the remaining quantity, appropriate notification of the end of life can be realized.

In a different embodiment, in a thirty-third aspect, a computer readable medium storing a computer-executable instruction is provided. Regarding the computer readable medium, a processor in the electric power source unit is made to perform the method in any one of the nineteenth to thirty-second aspects when the computer-executable instruction is executed.

Further, in a different embodiment, in a thirty-fourth aspect, an electric power source unit, which comprises a sensor for detecting a puff action performed by a user and a controller, for an inhaler is provided. Regarding the electric power source unit, the controller performs: measurement of detected-time that is a puff action period during that the detected puff action is continued; correction of the detected-time, by using a time correction model that is based on an atomization characteristic of an inhaled component source in the puff action; calculation of accumulated detected-time by accumulating the corrected detected-time; and estimation of a remaining quantity level of the inhaled component source, based on the accumulated detected-time.

According to the above electric power source unit, by flexibly adjusting detected-time of puff action performed by a user, appropriate accumulated detected-time can be estimated and accuracy of estimation of the remaining quantity level of the inhaled component source can be improved. Further, appropriate grasping and notifying of the remaining quantity can be realized.

An electric power source unit in a thirty-fifth aspect comprises the electric power source unit in the thirty-fourth aspect, and the estimation of the remaining quantity level includes judging that shortage in the remaining quantity of the inhaled component source has occurred, when the accumulated detected-time has reached predetermined threshold time. By applying judgment of occurrence of shortage in the remaining quantity, appropriate detection of the end of life can be realized.

An electric power source unit in a thirty-sixth aspect comprises the electric power source unit in the thirty-fifth aspect, and further comprises a notifier, and the controller makes the notifier operate to provide notification representing shortage in the remaining quantity, in response to the judgement of occurrence of shortage in the remaining quantity. By applying the construction for notifying shortage in the remaining quantity, appropriate notification of the end of life can be realized.

An electric power source unit in a thirty-seventh aspect comprises the electric power source unit in any one of the thirty-fourth to thirty-sixth aspects, and the time correction model is defined as a function of the puff action period and a puff action interval between two successive puff actions. By the above construction, it becomes possible to construct an appropriate time correction model, and further improve accuracy of estimation of the remaining quantity level of the inhaled component source.

BRIEF DESCRIPTION OF DRAWINGS

Fig. 1A is a schematic block diagram of a construction of an inhaler.
Fig. 1B is a schematic block diagram of a construction of an inhaler.
Fig. 2 is a schematic graph showing an example of relationship between the number of times of puffs and puff action periods.
Fig. 3 is a schematic graph showing an example of an atomization characteristic 1 of an aerosol source.
Fig. 4 is a schematic graph showing an example of an atomization characteristic 2 of an aerosol source.
Fig. 5A is a schematic graph showing an example of the atomization characteristic 1 of an aerosol source.
Fig. 5B is a schematic graph showing an example of a time correction model 1 based on the atomization characteristic 1.
Fig. 6A is a schematic graph showing an example of a time correction model 2 based on the atomization characteristic 2.
Fig. 6B is a schematic graph showing an example of a time correction model 2 based on the atomization characteristic 2.
Fig. 7 is a schematic block diagram of a construction of an electric power source unit according to a first embodiment.
Fig. 8 is a schematic flow chart of operation of the electric power source unit according to the first embodiment.
Fig. 9 is a schematic flow chart of operation of the electric power source unit according to the first embodiment.
Fig. 10 is a schematic graph showing an example of an atomization characteristic 1a of an aerosol source.
Fig. 11A is a schematic graph showing an example of a time correction model $1A_{ID}$ corresponding to the atomization characteristic 1a.
Fig. 11B is a schematic graph showing an example of a time correction model 1A based on the atomization characteristic 1a.
Fig. 12 is a schematic graph showing an example of an atomization characteristic 2a of an aerosol source.
Fig. 13A is a schematic graph showing an example of a time correction model $2A_{DIF}$ corresponding to the atomization characteristic 2a.
Fig. 13B is a schematic graph showing an example of a time correction model 2A based on the atomization characteristic 2a.
Fig. 14 is a schematic block diagram of a construction of an electric power source unit according to a second embodiment.
Fig. 15 is a schematic flow chart of operation of the electric power source unit according to the second embodiment.
Fig. 16 is a schematic flow chart of operation of the electric power source unit according to the second embodiment.
Fig. 17 is a schematic graph showing a different example of a time correction model 2B based on the atomization characteristic 2a.
Fig. 18 is a modification example of the schematic flow chart of operation of the electric power source unit according to the second embodiment.
Fig. 19 is a schematic block diagram of a construction of an electric power source unit according to a third embodiment.
Fig. 20 is a schematic flow chart of operation of the electric power source unit according to the third embodiment.

DESCRIPTION OF EMBODIMENTS

In the following description, respective embodiments of the present disclosure will be explained in detail with reference to the attached figures. It should be reminded that, although the embodiments of the present disclosure comprise an electronic cigarette and a nebulizer, the components are not limited to those explained above. The embodiments of the present disclosure may comprise various inhalers for generating aerosol or flavor-added aerosol inhaled by users. Further, the generated inhaled components include invisible vapor, in addition to aerosol.

< Basic Construction of Inhaler >

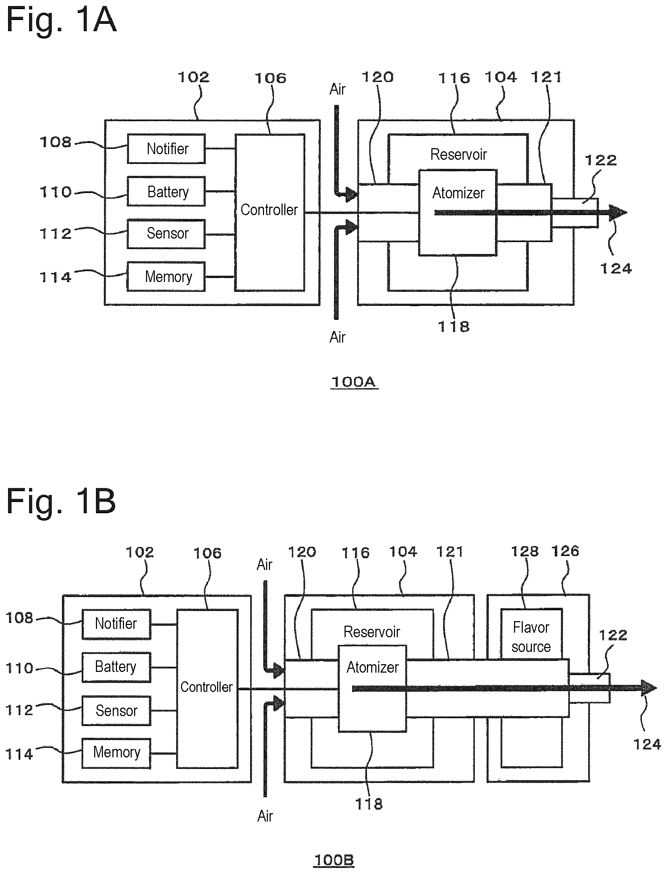

Fig. 1A is a schematic block diagram of a construction of an inhaler 100A according to each embodiment of the present disclosure. Fig. 1A is that schematically and conceptually showing respective components included in the inhaler 100A, and is not that showing precise positions, shapes, sizes, positional relationship, and so on of the respective components and the inhaler 100A.
As shown in Fig. 1A, the inhaler 100A comprises a first member 102 and a second member 104. As shown in the figure, in an example, the first member 102 may be an electric power source unit, and may comprise a controller 106, a notifier 108, a battery 110, a sensor 112, the memory 14. Also, in an example, the second member 104 may be a cartridge, and may comprise a reservoir 116, an atomizer 118, an air taking-in flow path 120, and an aerosol flow path 121, and a suction opening part 122.

Part of components included in the first member 102 may be included in the second member 104. Part of components included in the second member 104 may be included in the first member 102. The second member 104 may be constructed to be attachable/detachable to/from the first member 102. Alternatively, all components included in the first member 102 and the second member 104 may be included in a single same housing in place of the first member 102 and the second member 104.

An electric power source unit, which is the first member 102, comprises the notifier 108, the battery 110, the sensor 112, and the memory 114, and is electrically connected to the controller 106. In the above components, the notifier 108 may comprise a light emitting element such as an LED or the like, a display, a speaker, a vibrator, and so on. It is preferable that the notifier 108 provide a user with notification in various forms, by light emission, display, vocalization, vibration, or the like, or a combination thereof, as necessary. In an example, it is preferable that the remaining quantity level or the replacement timing of an inhaled component source included in the reservoir 116 in the second member 104 be notified in various forms.

The battery 110 supplies electric power to the respective components, such as the notifier 108, the sensor 112, the memory 114, the atomizer 118, and so on, in the inhaler 100A. Especially, the battery 110 supplies electric power to the atomizer 118 for atomizing an aerosol source in response to puff action of a user. The battery 110 can be connected to an external electric power source (for example, a USB (Universal Serial Bus) connectable charger) via a predetermined port (which is not shown in the figure) installed in the first member 102.

It may be constructed in such a manner that the battery 110 only can be detached from the electric power source unit 102 or the inhaler 100A, and can be replaced by a new battery 110. Also, it may be constructed in such a manner that the battery 110 can be replaced by a new battery 110, by replacing the whole electric power source unit by a new electric power source unit.

The sensor 112 comprises various kinds of sensors. For example, the sensor 112 may comprise a suction sensor such as a microphone condenser, for precisely detecting puff action of a user. Further, the sensor 112 may comprise a pressure sensor for detecting change in the pressure or a flow rate sensor for detecting a flow rate in the air taking-in flow path 120 and/or the aerosol flow path 121. Further, the sensor 112 may comprise a weight sensor for detecting the weight of a component such as the reservoir 116 or the like.

Further, the sensor 112 may be constructed to detect the height of a liquid surface in the reservoir 116. Further, the sensor 112 may be constructed to detect an SOC (State of Charge, charge state) of the battery 110, and a discharging state, an integrated current value, a voltage, or the like of the battery 110. The integrated current value may be obtained by using a current integration method, an SOC-OCV (Open Circuit Voltage, open circuit voltage) method, or the like. Further, the sensor 112 may comprise a temperature sensor for measuring the temperature of the controller 106. Further, the sensor 112 may be a manipulation button which can be manipulated by a user, or the like.

The controller 106 may be an electronic circuit module constructed as a microprocessor or a microcomputer. The controller 106 may be constructed to control operation of the inhaler 100A in accordance with computer-executable instructions stored in the memory 114. Further, the controller 106 may comprise a timer and may be constructed to measure, based on a clock, a desired period of time by use of the timer. In an example, the controller 106 may measure, by use of the timer, an action period during that puff action is being detected by the suction sensor and an action interval between successive puff actions.

The controller 106 reads data from the memory 114 and uses the data for controlling the inhaler 100A as necessary, and stores data in the memory 114 as necessary.

The memory 114 is a storage medium such as a ROM (Read Only Memory), a RAM (Random Access Memory), a flash memory, or the like. The memory 114 may store, in addition to computer-executable instructions such as those explained above, setting data and so on that are necessary for controlling the inhaler 100A and/or the electric power source unit 102, and may mainly be used by the controller 106. For example, the memory 114 may store various data such as methods for controlling the notifier 108 (modes of light emission, vocalization, vibration, etc., and so on), values detected by the sensor 112, information relating to an attached cartridge, history of heating relating to the atomizer 118, and so on.

Regarding the cartridge which is the second member 104, the reservoir 116 holds an aerosol source which is an inhaled component source. For example, the reservoir 116 comprises fibrous or porous material, and holds an aerosol source, which is in the form of liquid, by use of spaces between fibers or pores in the porous material. Cotton or glass fibers, or tobacco raw material, or the like, may be used as the above-explained fibrous or porous material. The reservoir 116 may be constructed as a tank for storing liquid. The aerosol source is liquid such as polyhydric alcohol, such as glycerin or propylene glycol, or water, or the like, for example.

In the case that the inhaler 100A is an inhaler for medical use, such as a nebulizer or the like, the aerosol source may also comprise a medicine that is to be inhaled by a patient. In a different example, the aerosol source may comprise a tobacco raw material or an extract originated from a tobacco raw material, which releases a fragrance-inhaling-taste component when it is heated. The reservoir 116 may have a construction which allows replenishment of a consumed aerosol source. Also, the reservoir 116 may be constructed in such a manner that the reservoir 116 itself is allowed to be replaced when the aerosol source is exhausted. Further, the aerosol source is not limited to that in a liquid form, and it may be solid. In the case that the aerosol source is solid, the reservoir 116 may be a hollow container which does not use fibrous or porous material, for example.

The atomizer 118 is constructed to generate aerosol from an aerosol source. In more detail, the atomizer 118 generates aerosol by atomizing or vaporizing an aerosol source. In the case that the inhaler 100A is a medical inhaler such as a nebulizer or the like, the atomizer 118 generates aerosol by atomizing or vaporizing an aerosol source including a medicine.

When puff action is detected by the sensor 112, the atomizer 118 generates aerosol by receiving supply of electric power from the battery 110. For example, a wick (which is not shown in the figure) may be installed for connection between the reservoir 116 and the atomizer 118. In the above case, a part of the wick extends to the inside of the reservoir 116 and is in contact with the aerosol source. The other part of the wick extends toward the atomizer 118. The aerosol source is sent from the reservoir 116 to the atomizer 118 by capillary effect in the wick.

In an example, the atomizer 118 comprises a heater which is electrically connected to the battery 110. The heater is arranged to be in contact with or to be positioned close to the wick. When a puff action is detected, the controller 106 controls the heater in the atomizer 118 to heat an aerosol source, which is conveyed via the wick, to thereby atomize the aerosol source. The other example of the atomizer 118 may be an ultrasonic-type atomizer which atomizes the aerosol source by ultrasonic vibration.

The air taking-in flow path 120 is connected to the atomizer 118, and the air taking-in flow path 120 leads to the outside of the inhaler 100A. The aerosol generated in the atomizer 118 is mixed with air that is taken via the air taking-in flow path 120. The fluid mixture comprising the aerosol and the air is sent to the aerosol flow path 121, as shown by an arrow 124. The aerosol flow path 121 has a tubular structure for sending the fluid mixture comprising the air and the aerosol, that is generated in the atomizer 118, to the suction opening part 122.

The suction opening part 122 is constructed in such a manner that it is positioned at an end of the aerosol flow path 121, and makes the aerosol flow path 121 be opened toward the outside of the inhaler 100A. A user can take air including the aerosol into the user's mouth by holding the suction opening part 122 in the user's mouth and performing a suction action.

Fig. 1B is a schematic block diagram of a construction of an inhaler 100B according to respective embodiments of the present disclosure. As shown in Fig. 1B, the inhaler 100B comprises a third member 126, in addition to the constructions included in the inhaler 100A in Fig. 1A. The third member 126 may be a capsule, and may comprise a flavor source 128. For example, in the case that the inhaler 100B is an electronic cigarette, the flavor source 128 may comprise a fragrance-inhaling-taste component included in tobacco. As shown in the figure, the aerosol flow path 121 extends across the second member 104 and the third member 116. The suction opening part 122 is installed in the third member 126.

The flavor source 128 is a component for adding flavor to aerosol. The flavor source 128 is positioned in the middle of the aerosol flow path 121. The fluid mixture comprising the air and the aerosol generated by the atomizer 118 (it should be reminded that the fluid mixture may simply be referred to as aerosol, hereinafter) flows to the suction opening part 122 through the aerosol flow path 121. In this manner, in the point of view of the flow of the aerosol, the flavor source 128 is arranged in a position downstream the atomizer 118. In other words, in the aerosol flow path 121, the position of the flavor source 128 is closer to the suction opening part 122 than the position of the atomizer 118.

Thus, the aerosol generated in the atomizer 118 passes through the flavor source 128 and thereafter arrives at the suction opening part 122. When the aerosol passes through the flavor source 128, fragrance-inhaling-taste components included in the flavor source 128 are added to the aerosol. For example, in the case that the inhaler 100B is an electronic cigarette, the flavor source 128 may be that which originates from tobacco, such as shredded tobacco, a product which is made by processing tobacco raw material to have a granular form, a sheet form, or a powder form, or the like.

The flavor source 128 may be that which does not originate from tobacco, such as that made by use of a plant other than tobacco (for example, mint, a herb, and so on). For example, the flavor source 128 comprises a nicotine component. The flavor source 128 may comprise a flavor component such as menthol or the like. In addition to the flavor source 128, the reservoir 116 may also have a material comprising a fragrance-inhaling-taste component. For example, the inhaler 100B may be constructed in such a manner that the flavor source 128 holds flavor material which originates from tobacco and the reservoir 116 includes flavor material which does not originate from tobacco.

A user can take air including the aerosol, to which the flavor has been added, into the mouth by holding the suction opening part 122 in the user's mouth and performing a suction action.

< First Embodiment >

The electric power source unit 102 installed in each of the inhalers 100A and 100B (hereinafter, they may collectively be referred to as an "inhaler 100") according to a first embodiment of the present disclosure is controlled by the controller 106 by using various methods. In the following description, a method for operating an electric power source unit 102 in an inhaler according to the first embodiment of the present disclosure will be explained in detail.

(1) Basic Method for Estimating Remaining Quantity Level of Inhaled Component Source

For providing a user with a comfortable suction experience and continuously providing the user with sufficient flavor-added aerosol, it is preferable to appropriately grasp the remaining quantity level (or the consumption level) of the aerosol source and/or the flavor source 128 stored in the reservoir 116 and/or the capsule. Further, it is preferable to urge a user to replace the cartridge and/or the capsule at timing when it is judged that there is no remaining quantity. In an example for appropriately grasping the remaining quantity level, it is preferable that the controller 106 use accumulated time that has been spent by a user for performing puff action, specifically, the controller 106 perform operation based on whether the accumulated time has reached a predetermined threshold value.

For example, regarding an aerosol source in the reservoir 116 held in the cartridge, the controller 106 may judge that the aerosol source has been exhausted, at the time when the accumulated time of puff action reaches a predetermined upper limit, after attaching of the cartridge. Regarding the cartridge, the above predetermined upper limit is 1000 seconds, for example. In the inhaler 100B, regarding a flavor source held in the capsule, it may be judged, in a manner similar to the above manner, that the flavor source has been exhausted, at the time when the accumulated time of puff action reaches a predetermined upper limit, after attaching of the capsule. Regarding the capsule, the above predetermined upper limit is 100 seconds, for example. Further, in the case that it is judged that the aerosol source and/or the flavor source have/has been exhausted, it is preferable to urge a user to replace the cartridge and/or the capsule which hold/holds the aerosol source and/or the flavor source.

The above matters are based on the technical idea that, during the period when the inhaler is accepting puff action stably, the quantities/quantity of consumption of the cartridge and/or the capsule are/is substantially proportional to an accumulated value of puff action periods. Further, by using the above as a premise, it becomes possible to define, by using the accumulated time as a parameter, the quantities/quantity of consumption of the aerosol source and/or the flavor source, and measure them/it easily.

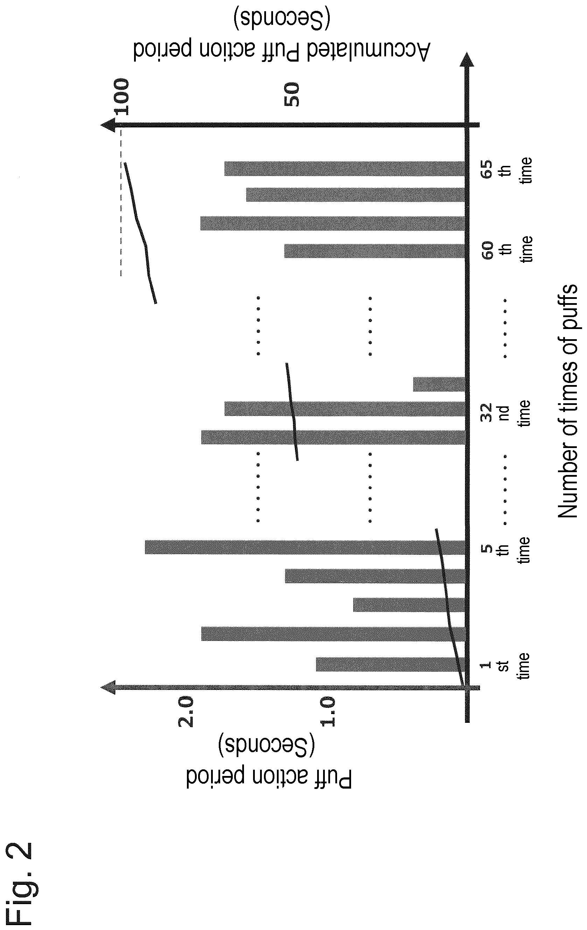

Fig. 2 is a schematic graph showing an example of relationship between the number of times of puffs, puff action periods, and accumulated puff action periods, relating to consumption of a flavor source held in a capsule. The horizontal axis represents the number of times of puffs (n-th time) after attaching of a new capsule. Further, the left vertical axis represents a puff action period (in seconds) per single puff action, and the right vertical axis represents an accumulated puff action period (in seconds). Still further, bars in the bar graph represent puff action periods (in seconds) measured with respect to respective numbers of times of puffs, and the line graph represents the accumulated puff action periods (in seconds).

In the example shown in the figure, a single puff action period is that in the range from 0.3 seconds to 2.4 seconds, approximately, and 65 times of puff actions are required for incrementing the accumulated puff action period (in seconds) to become 100 seconds. That is, regarding the capsule, in the case that the predetermined upper limit threshold value relating to the accumulated time of puff action has been set to 100 seconds, it is preferable to judge, in response to the 65th puff action, that the flavor source has been exhausted. Further, it is preferable that the consumption level be calculated based on the value of the accumulated puff action period. For example, in the case that the value of the accumulated puff action period until the 32th puff action is 50 seconds, it is preferable that the consumption level be inferred as 50 % (50 seconds / 100 seconds * 100). It should be reminded that, regarding the inhaler 100B in which the cartridge 104 and the capsule 126 are constructed by use of different components, the matter that "the upper limit threshold value of the accumulated time of puff action is 100 seconds" is based on the technical idea that the total quantity of aerosol (flavor is added thereto by the flavor source), that has been generated by atomizing the aerosol source in response to puff action performed for 100 seconds, that is the accumulated time, and has passed through the flavor source, is the quantity that is sufficient to make the flavor source reach the end of its life. In this regard, "the flavor source reaches the end of its life" means that the state becomes that wherein sufficient flavor cannot be added to aerosol that is generated by consuming and atomizing the aerosol source.

Based on profound knowledge obtained by the inventors, an applied method for estimating a remaining quantity level, that is more precise than the above-explained basic method for estimating a remaining quantity level, will be suggested. According to an experiment performed by the inventors, it has been ascertained that, in the case that an estimation method including a process for associating an accumulated puff action period with a remaining quantity level is used, estimation accuracy can be further improved by incorporating, in the estimation method, a control technique for correcting, based on an atomization characteristic of an aerosol source, a value of a puff action period.

(2) Applied Method for Estimating Remaining Quantity Level

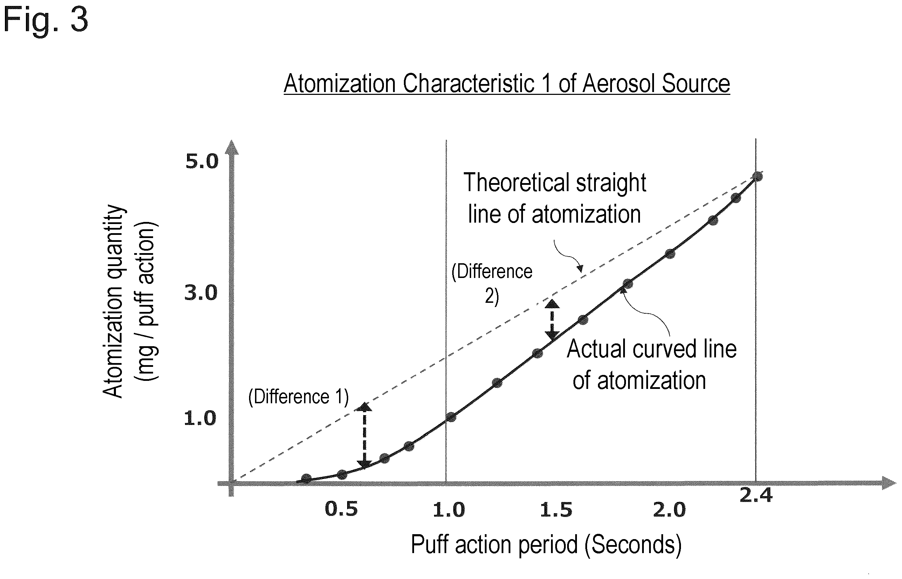

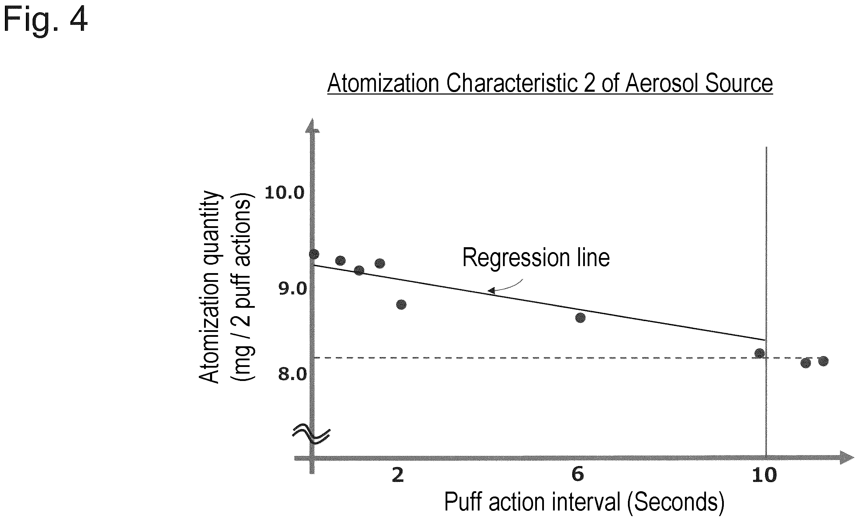

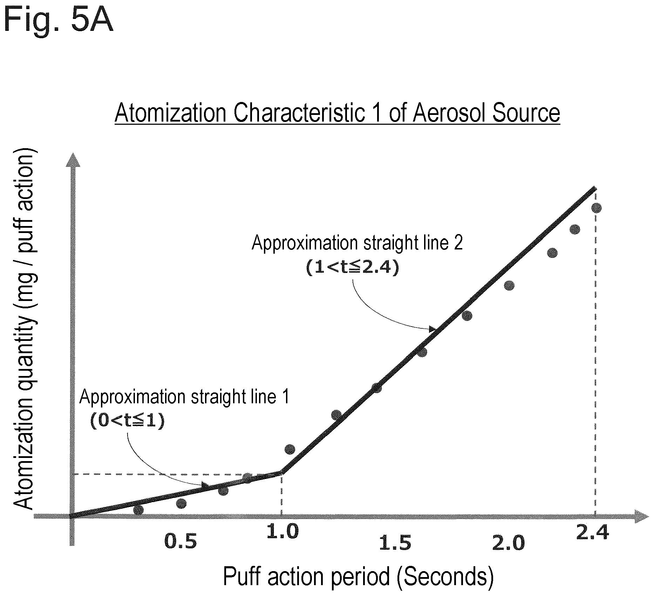

Each of Fig. 3 and Fig. 4 is a schematic graph showing an atomization characteristic of an aerosol source, relating to puff action performed by a user by using the inhaler 100. By using each of the above graphs as the basis, an atomization characteristic of an aerosol source in an atomization phenomenon in the inhaler 100 can be specified. Further, Fig. 5A to Fig. 6B are schematic graphs showing time correction models that are designed based on the atomization characteristics of the aerosol source, according to the first embodiment.

(2-1) Atomization Characteristics of Aerosol Source

The graph in Fig. 3 relates to an atomization phenomenon in the inhaler 100 using a sample flavor source, and shows an example of relationship between a puff action period and an atomization quantity per single puff action. The horizontal axis represents a puff action period (in seconds) per single puff action. Specifically, a puff action period is a period from a start of a puff action to an end of the puff action. The vertical axis represents an atomization quantity per single puff action, that is, a consumption quantity (mg / puff action) of the aerosol source. Specifically, the atomization quantity is a quantity calculated by subtracting, from the weigh of the aerosol source at the time of a start of a puff action, the weigh of the aerosol source at the time of an end of the puff action.
In more detail, regarding the puff action period represented by the horizontal axis, data thereof can be obtained by detecting the time of a start of a puff action and the time of an end of the puff action by using a suction sensor and a timer, and measuring a continuous period between the start time and the end time of the puff action by the timer. Further, regarding the atomization quantity represented by the vertical axis, the data thereof can be obtained by measuring the weigh of the aerosol source at the time of a start of a puff action and the weigh of the aerosol source at the time of an end of the puff action by using, for example, a weight sensor, and calculating a difference in the weight.

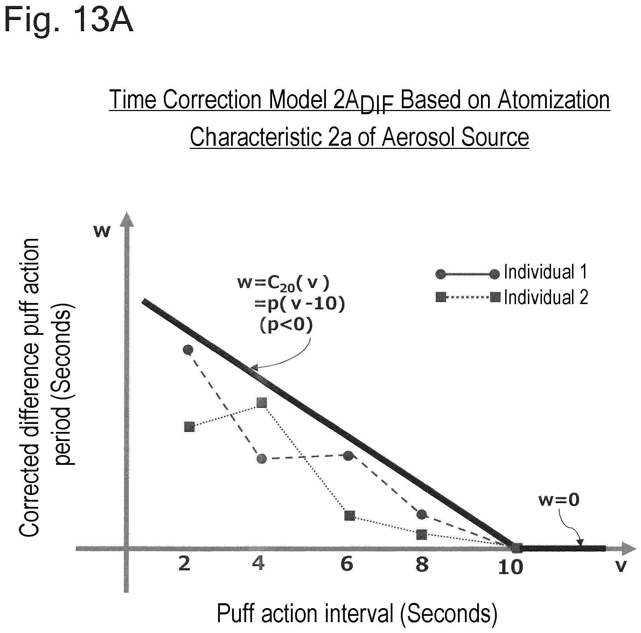

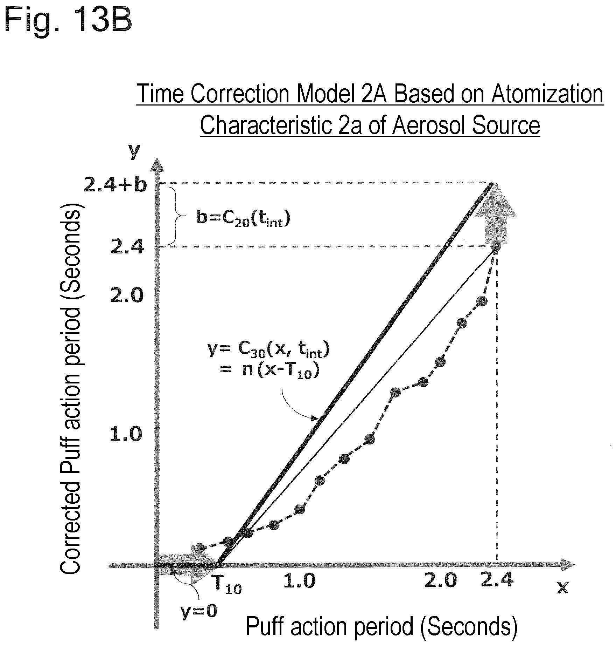

In Fig. 3, 13 sample points that were measured in an atomization phenomenon are plotted. Also, an actual atomization curve line, that is based on the above 13 sample points, and a theoretical atomization straight line are shown. The theoretical atomization straight line is drawn in such a manner that an origin and a sample point (2.4 seconds, the longest puff action period) that is the farthest from the origin are connected by the straight line. The above is based on the idea that the atomization quantity increases in proportion to the suction time.
As shown in the figure, when the actual atomization curve line and the theoretical atomization straight line are compared, it can be understood that there is separation between them. Specifically, unlike the theoretical atomization straight line, the puff action period and the actual atomization quantity are not proportional to each other in the actual atomization curve line. Especially, regarding each of the puff action periods having lengths shorter than approximately 2.4 seconds, it can be seen, at least, that the actual atomization quantity is smaller than the theoretical atomization quantity. In more detail, the different between the above two quantities becomes larger as the time of the puff action period becomes longer, until the time reaches approximately 1 second (difference 1), and, thereafter, becomes smaller as the time of the puff action period further becomes longer (difference 2). The above matter occurs due to certain rise time that is required and so on, wherein the rise time is that from the time when heating operation of a heater is started at a start of a puff action to the time when the temperature reaches preferred temperature that makes it possible to perform atomization.
The graph in Fig. 4 relates to an atomization phenomenon in the inhaler 100 using a sample flavor source, and shows an example of relationship between an action interval between two successive puff actions and an atomization quantity atomized through the two successive puff actions. The horizontal axis represents a puff action interval (in seconds) between two successive puff actions. Specifically, a puff action interval is a period from an end of a first puff action to a start of a next, i.e., a second puff action. The vertical axis represents an atomization quantity, that is, a consumption quantity (mg / two puff actions), of an aerosol source atomized through two successive puff actions. Specifically, the atomization quantity is a quantity calculated by subtracting, from the weigh of the aerosol source at the time of a start of a first puff action, the weigh of the aerosol source at the time of an end of a second puff action.
In more detail, regarding the puff action interval, data thereof can be obtained by detecting the time of a start of a puff action and the time of an end of a puff action by using a suction sensor, and measuring time between a period from the time of an end of the first puff action and the time of a start of the second puff action by the timer. Further, regarding the atomization quantity, the data thereof can be obtained by measuring the weigh of the aerosol source at the time of a start of the first puff action and the weigh of the aerosol source at the time of an end of the second puff action by using, for example, a weight sensor, and calculating a difference in the weight.

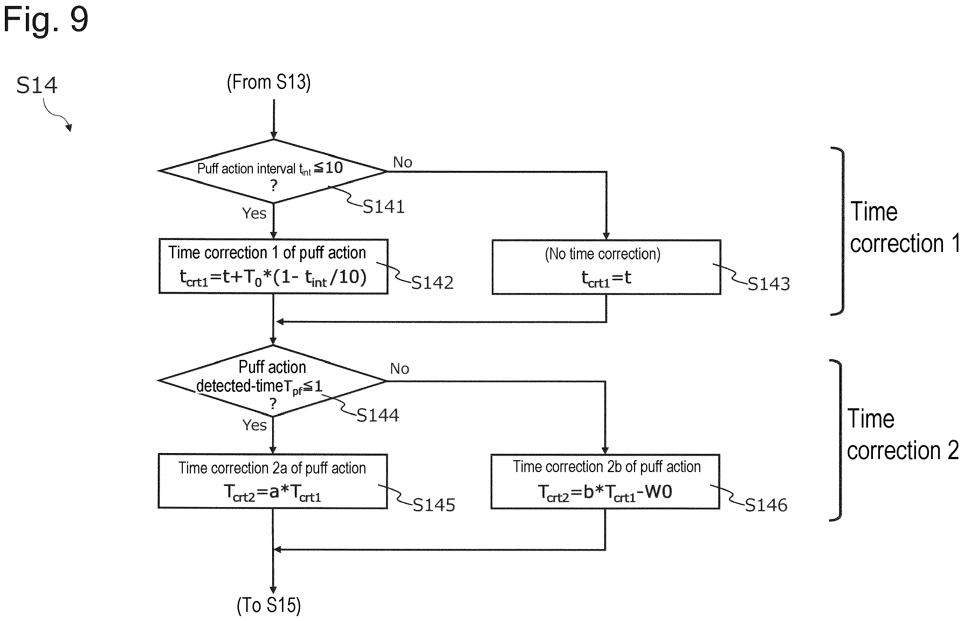

In Fig. 4, 9 sample points that were measured in an atomization phenomenon are plotted. Further, regarding 7 pieces of data relating to puff action intervals, each thereof is approximately equal to or shorter than 10 seconds, a regression line is shown; wherein the regression line is based on linear regression that uses a puff action interval as an explanatory variable and an atomization quantity as an objective variable. As shown in the figure, there is negative correlation in the present case. That is, in an actual atomization phenomenon, the atomization quantity of the atomized aerosol source becomes larger (approximately 8.8 mg - 9.3 mg) as the puff action interval becomes shorter. On the other hand, regarding the case wherein each of puff action intervals is longer than approximately 10 seconds, the atomization quantity of the aerosol source is generally constant and stabilized (approximately 8.1 mg: the dotted line). The above matter in the atomization phenomenon in the inhaler 100 occurs due to rise time, that is shorter than usual rise time, of the heater when a puff action following a previous puff action is started in the condition that the puff action interval is equal to or shorter than 10 seconds, and so on; wherein the reason that the rise time is shortened is that the heater heated during the previous puff action is not cooled sufficiently and residual heat remains in the heater. As a result, the atomization quantity becomes larger compared with those in the stable state wherein each puff action interval is longer than 10 seconds.

In this manner, regarding the atomization phenomenon of an aerosol source, it is preferable to specify two atomization characteristics of the aerosol source in advance, and reflect them to controlling of estimation of the remaining quantity level. Specifically, by incorporating the control technique for correcting a value of a puff action period based on the above two atomization characteristics, it becomes possible to further improve accuracy of estimation of the remaining quantity of the aerosol source and/or the remaining quantity of the flavor source. In the following description, the two atomization characteristics (atomization characteristics 1 and 2) of the aerosol source are summarized.

[ Atomization characteristic 1 ] The atomization characteristic 1 is defined based on relationship between sample action periods of puff action and atomization quantities. In the present case, an actual atomization quantity of an aerosol source is lower than a theoretical atomization quantity. Further, regarding the case that a puff action period is approximately equal to or shorter than 1 second, the different between the theoretical value and the measured value becomes larger as the time of the puff action period becomes longer. On the other hand, regarding the case that a puff action period is approximately equal to or longer than 1 second, the different between the theoretical value and the measured value becomes smaller as the time of the puff action period becomes longer. If an actual value of a puff action period is applied as it stands to estimation of a remaining quantity level, a remaining quantity larger than an actual remaining quantity may be estimated; so that it is preferable to correct the value of the puff action period to make it somewhat smaller, and estimate the remaining quantity level of the aerosol source.

Similarly, as explained above, an actual atomization quantity of an aerosol source is lower than a theoretical atomization quantity. That is, regarding the case of the inhaler 100B in which the cartridge 104 and the capsule 126 are constructed by use of different components, an actual quantity of aerosol that passes through the flavor source held in the capsule 126 is smaller than a theoretical quantity of aerosol. That is, by adopting the construction for correcting the value of a puff action period to make it somewhat smaller and estimating a remaining quantity level of the flavor source, accuracy of estimation of the remaining quantity of the aerosol source and the remaining quantity of the flavor source can be further improved.

[ Atomization characteristic 2 ] The atomization characteristic 2 is defined based on relationship between sample action intervals between respective two successive puff actions and atomization quantities of an aerosol source (Fig. 4). In the present case, there is negative correlation between puff action intervals and atomization quantities of the aerosol source in the case that each of the puff action intervals is equal to or shorter than 10 seconds, so that the atomization quantity of the aerosol source becomes smaller as the puff action interval becomes longer. That is, regarding the case that the puff action interval is equal to or shorter than 10 seconds, if an actual value of a puff action period is applied as it stands to estimation of a remaining quantity level, a remaining quantity smaller than an actual remaining quantity may be estimated. Thus, it is preferable to correct the value of the puff action period to make it somewhat larger, and estimate the remaining quantity level of the aerosol source.

Similarly, as explained above, in the case that each of puff action intervals is equal to or shorter than 10 seconds, if an actual value of a puff action period is applied as it stands to estimation of a remaining quantity level, a remaining quantity smaller than an actual remaining quantity may be estimated. That is, in the case of the inhaler 100B in which the cartridge 104 and the capsule 126 are constructed by use of different components, an actual quantity of aerosol that passes through the flavor source held in the capsule 126 may be estimated as that smaller than the actual quantity of aerosol. Thus, by adopting the construction for correcting the value of the puff action period to make it somewhat lager and estimating a remaining quantity level of the flavor source, accuracy of estimation of the remaining quantity of the aerosol source and the remaining quantity of the flavor source can be further improved.

The electric power source unit in the inhaler 100 according to the first embodiment is constructed to accurately estimate a remaining quantity level, through dynamic correction of detected-time, that is the time during that a detected puff action is continued, in accordance with the atomization characteristics 1 and 2 of an aerosol source relating to puff action. That is, appropriate estimation of the remaining quantity levels/level of the flavor source and/or the aerosol source is realized, by estimating a puff action period and an accumulated puff action period that are more accurate, compared with detected-time of an actually detected puff action. By using the above technique, appropriate estimation of a consumption level, judgment with respect to replacement, and notification relating to a cartridge and/or a capsule is realized.

(2-2) Time Correction Models Generated Based on Atomization Characteristics

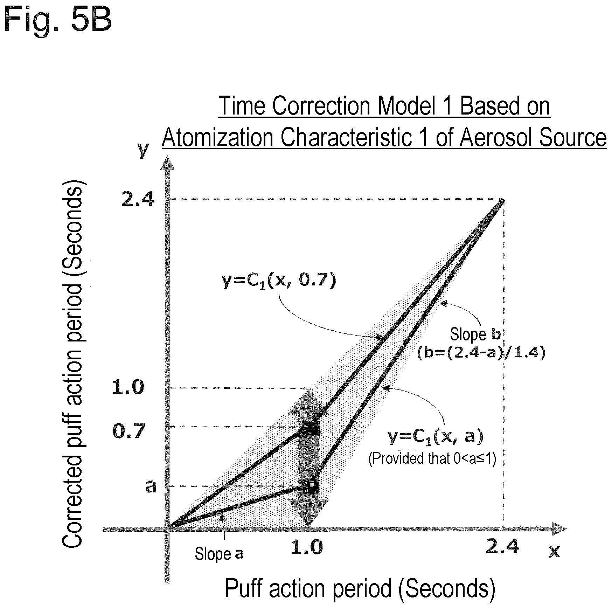

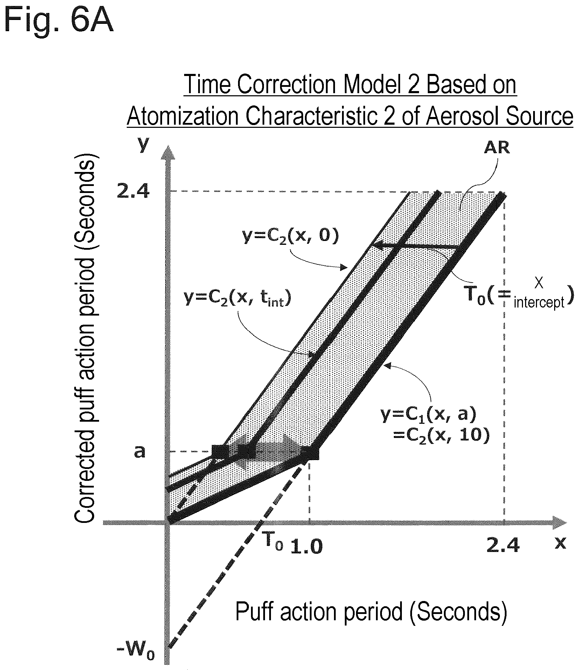

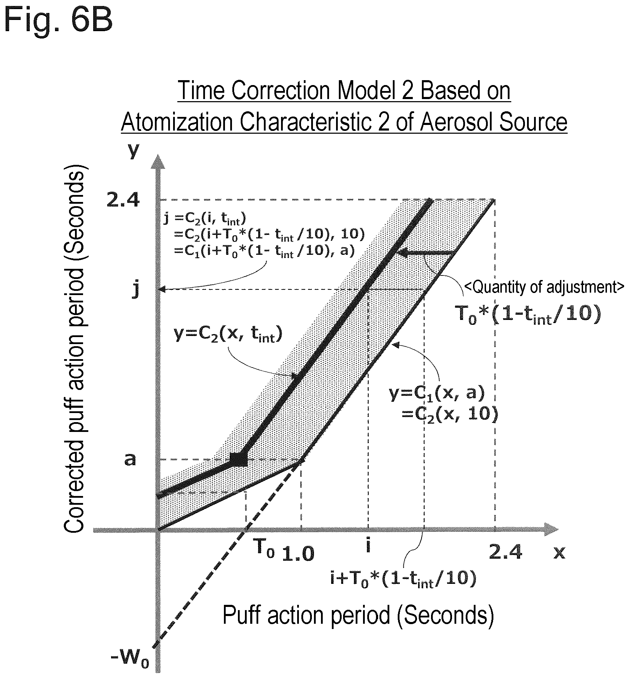

With reference to Fig. 5A to Fig. 6B, methods for generating time correction models 1 and 2 for correcting detected-time of detected puff actions, according to the atomization characteristics 1 and 2 of an aerosol source, will be explained in detail. Each of Fig. 5A and Fig. 5B is a schematic figure for explaining a time correction model 1 based on the atomization characteristic 1. Also, Each of Fig. 6A and Fig. 6B is a schematic figure for explaining a time correction model 2 based on the atomization characteristic 2.

[ Time Correction Model 1 Based on Atomization Characteristic 1 ]

In Fig. 5A, 13 sample points of the atomization quantities and the puff action periods shown in the graph in Fig. 3 are used. In the present case, in accordance with the atomization characteristic 1, two approximation straight lines are shown in the sections before and after the puff action period of 1.0 second. As shown in the figure, it will be understood by a person skilled in the art that the atomization characteristic 1 of the aerosol source can be expressed qualitatively in an appropriate manner, by performing approximation by using two contiguous straight lines (approximation straight lines 1 and 2) in the section between the point where the puff action period is 0 second and the point where the puff action period is 1.0 second and the section between the point where the puff action period is 1.0 second and the point where the puff action period is 2.4 seconds. Especially, it is recognized based on the atomization characteristic 1 that the slope of the approximation straight line 1 is smaller than that of the approximation straight line 2.

The time correction model 1 shown in Fig. 5B is generated based on the atomization characteristic 1 in Fig. 5A. In the graph shown in Fig. 5B, the horizontal axis (x axis) represents a puff action period (in seconds) and the vertical axis (y axis) represents a corrected puff action period (in seconds) relating to the puff action period. In accordance with the time correction model 1 shown in the figure, the puff action period is corrected to the corrected puff action period. Specifically, similar to the case of Fig. 5A, it is preferable that the atomization quantities be defined by using two linear functions in the sections before and after the point where the puff action period is 1.0 second. Further, in accordance with the atomization characteristic 1 of the aerosol source, a puff action period is corrected in such a manner that a to-be-consumed atomization quantity is underestimated, that is, a corrected puff action period is made to be shorter than an actual puff action period. In more detail, regarding the time correction model 1, it is set in such a manner that, when the puff action period is 2.4 seconds, the corrected puff action period is 2.4 seconds (the correction factor is 1), and, when the puff action period is 1.0 second, the value (a) of the corrected puff action period takes a value between 0 second to 1.0 second. Thereafter, by connecting three points, specifically, coordinates (0, 0), (1.0, a), and (2.4, 2.4), by straight lines, the time correction model 1 is generated.

Specifically, the time correction model 1 is represented as a function of variables "x" and "a" shown below:

$$y = C_1 (x, a)$$

(Provided that 0<a<1)

In this regard, in Fig. 5B, an example relating the case when a=0.7, i.e., $y=C_1(x, 0.7)$, is shown.

In more detail, the function $y=C_1(x, a)$ of the time correction model 1 is represented by the following two linear functions (Formula 1):

-    The case of $0 < x \leq 1$

$$y = a * x$$

-    The case of $1 < x \leq 2.4$

$$y = b * x - W_0$$
$$= ((2.4 - a) / 1.4) * x - (2.4 / 1.4) * (1 - a)$$

(Provided that $0 < a < 1$)

The value of "a" is determined in advance in the range of 0<a<1. Further, "b" is a slope of a straight line formed by connecting coordinates (1.0, a) and (2.4, 2.4), that is, b=(2.4-a)/1.4; and a<b. In this regard, Wo is a y intercept of the linear function representing the straight line obtained by connecting two points, specifically, coordinates (1.0, a) and (2.4, 2.4). Specifically, it is represented as y=bx-$W_0$=((2.4-a)/1.4)x-$W_0$; thus, by performing substitution of the value (1.0, a) and arranging a=((2.4-a)/1.4)-$W_0$, the following representation $W_0$=(2.4/1.4)*(1-a) can be obtained. By using two linear functions in relation to sections before and after X=1 as explained above, a puff action period can be corrected and a corrected puff action period can be calculated, in accordance with the atomization characteristic 1.

[ Time Correction Model 2 Based on Atomization Characteristic 2 ]

Fig. 6A shows a time correction model 2 wherein the atomization characteristic 2 of the aerosol source shown in Fig. 4 is further applied to the time correction model 1 shown in Fig. 5B. In Fig. 6A, a function $C_2$(x, tint) in the time correction model 2 is generated by adjusting the function $C_1$(x, a) in the time correction model 1. Specifically, in accordance with the atomization characteristic 2 of the aerosol source, a puff action period is corrected in such a manner that a to-be-consumed atomization quantity, in the case that a puff action period is equal to or shorter than 10 seconds, is estimated as that larger than an actual atomization quantity, that is, a corrected puff action period is made to be longer than an actual puff action period.

The function $C_2$(x, tint) in the time correction model 2 is constructed as a function that uses a puff action period (x) and a puff action interval (y) as two variables. y=$C_1$(x, a) shown in Fig. 5B corresponds to the case when a puff action interval is 10 seconds, i.e., the function y=$C_2$(x,10). The above means that y=$C_1$(x, a)=$C_2$(x,10). This is because the function y=$C_2$(x,10) is a standard function before adjustment in the time correction model 2, and y=$C_1$(x, a) can be applied as it stand.

In more detail, as explained above, the function y=$C_2$(x,10) in the time correction model 2 is represented by the following two linear functions (Formula 2), based on Formula 1:

-    The case of $0 < x \leq 1$

$$y = C_2 (x, 10) = a * x$$

-    The case of $1 < x \leq 2.4$

$$y = C_2 (x, 10) = b * x - W_0$$
$$= ((2.4 - a) / 1.4) * x - (2.4 / 1.4) * (1 - a)$$

(Provided that $0 < a < 1$)

In this regard, by performing substitution of y=0 in Formula 2, an x intercept (To) of the linear function in the case of $1<x\leq2.4$ is represented as the following formula (Formula 3):

$$T_0 = (2.4 * (1 - a)) / (2.4 - a)$$

On the other hand, in the time correction model 2, the function in the case that a puff action interval tint is 0 second, i.e., y=$C_2$(x, 0), can be obtained by using the above $T_0$. Specifically, as shown in the figure, it is preferable that $C_2$(x, 0) be moved in parallel in the -x direction by $T_0$.

The function y=$C_2$(x, $t_{int}$) in the time correction model 2 can be defined in a dotted area AR shown in Fig. 6A, that is formed based on $C_2$(x,0) wherein the puff action interval tint is 0 second and $C_2$(x,10) wherein the puff action interval tint is 10 second. Specifically, y=$C_2$(x, tint) can be defined by performing adjustment in such a manner that $C_2$(x, 10) is moved in parallel in a -x axis direction toward $C_2$(x,0) by a certain quantity (this will be explained later).

In this regard, regarding each of cases wherein the puff action period (tint) is longer than 10 seconds, the function $C_2$(x,10), wherein the puff action interval tint is 10 second, may be shared. This is because, according to the atomization characteristic 2 of the aerosol source, it can be regarded that no negative correlation exists between a puff action interval and an atomization quantity relating to puff action, in the case that the puff action interval is longer than 10 seconds (the dotted line in Fig. 4), and, therefore, it is not necessary to perform adjustment based on the time correction model 2.

With reference to Fig. 6B, the function y=$C_2$(x, $t_{int}$) in the time correction model 2 will be explained further in detail. As shown in the figure, a corrected puff action period "j" relating to a puff action period "i" is obtained by moving the function $C_2$(x,10) in parallel in a -x axis direction by a quantity $T_0$*(10-$t_{int}$)/10=$T_0$*(1-$t_{int}$/10), and performing substitution of x=i in the time-adjusted function y=$C_2$(x, $t_{int}$). That is, $T_0$*(1-$t_{int}$/10) is an adjustment quantity. In this regard, the adjustment quantity corresponds to the quantity corresponding the $(10-t_{int})$ part, in terms of the ratio of (tint) versus $(10-t_{int})$, in the value of $T_0$ when the value is divided in proportion to the ratio of (tint) versus $(10-t_{int})$.

Specifically, the function $y=C_2(x, tint)$ in the time correction model 2 is represented by the following formulas (Formula 4):

$$y = C_2 (x, t_{int})$$
$$= C_2 (x+T_0*(1-t_{int}/10), 10)$$
$$= C_1 (x+T_0*(1-t_{int}/10), a)$$

In more detail, the function $y=C_2(x, tint)$ in the time correction model 2 is represented by the following two linear functions (Formula 5), based on Formulas 1 and 4:

- The case of $0 < x \leq 1$

$$y = a * (x + T_0 * (1 - t_{int} / 10))$$

- The case of $1 < x \leq 2.4$

$$y = b * (x + T_0 * (1 - t_{int} / 10)) - W_0$$
$$= ((2.4 - a) / 1.4) * (x + T_0 * (1 - t_{int} / 10)) - (2.4 / 1.4) * (1 - a)$$
$$(Provided\ that\ 0 < a < 1)$$

In this regard, $T_0$ is that represented by Formula 3.

As explained above, by defining the time correction models 1 and 2 based on the atomization characteristics 1 and 2 of the aerosol source, the corrected puff action period y can be obtained finally from the puff action period x, the puff action interval $t_{int}$, and the constant a, as shown by Formula 5. That is, operation for detecting, by the sensor 112, each puff action performed by a user using the inhaler 100, measuring detected-time, that is the time during that the detected puff action is being continued, measuring a puff action interval between two successive puff actions, and substituting them for the puff action period x and the puff action interval tint in Formula 5 may be performed. In this regard, the constant "a" may be set appropriately in advance to have a value within the range of 0<a<1, to correspond to the device characteristic of the inhaler 100, at the time of designing thereof.

(3) Functional Block Diagram Relating to Estimation of Remaining Quantity Level of Inhaled Component Source by Electric Power Source Unit

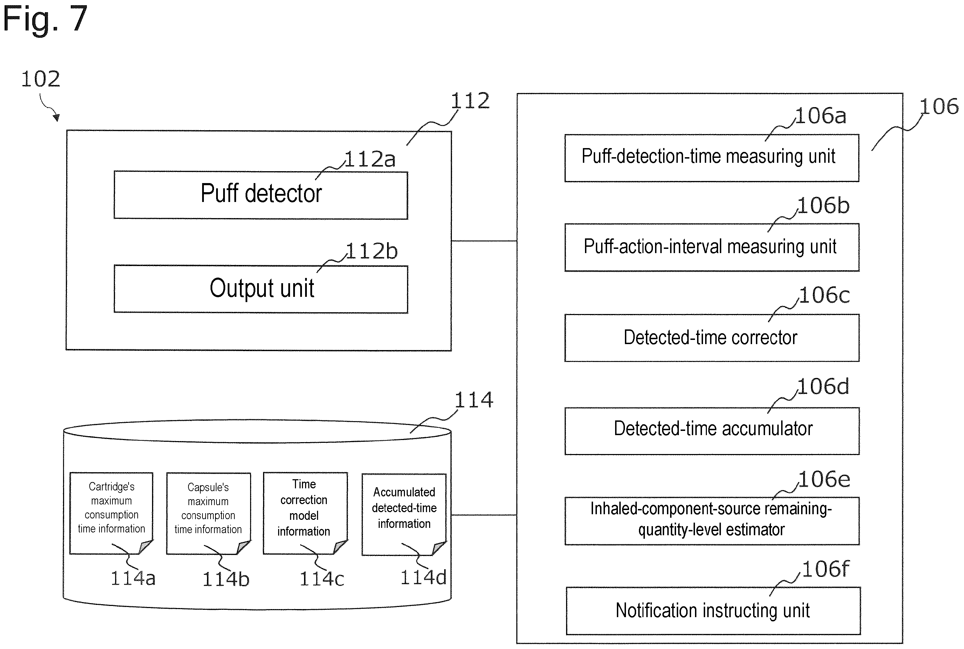

Fig. 7 relates to an electric power source unit 102 which is a component of the inhaler 100 according to the first embodiment, and shows examples of main functional blocks implemented by the controller 106 and the sensor 112, and examples of main pieces of information stored in the memory 114. The controller 106 controls, in cooperation with the sensor 112 and the memory 114, various kinds of operation relating to estimation of the remaining quantity levels/level of the flavor source and/or the aerosol source. Examples of functional blocks of the controller 106 comprise a puff-detection-time measuring unit 106a, a puff-action-interval measuring unit 106b, a detected-time corrector 106c, a detected-time accumulator 106d, an inhaled-component-source remaining-quantity-level estimator 106e, and a notification instructing unit 106f. Examples of functional blocks of the sensor 112 comprise a puff detector 112a and an output unit 112b. An example of information stored in the memory 114 comprises time information such as cartridge's maximum consumption time information 114a, capsule's maximum consumption time information 114b, time correction model information 114c, accumulated detected-time information 114d, and so on.

Regarding the functional blocks of the controller 106, the puff-detection-time measuring unit 106a measures detected-time (a period) of puff action detected by the puff detector 112a. Specifically, the puff-detection-time measuring unit 106a may continuously measure, by a timer, a period between the start time and the end time of a puff action detected by the puff detector 112a. The puff-action-interval measuring unit 106b measures an action interval between two successive puff actions. Specifically, the puff-action-interval measuring unit 106b may continuously measure, with respect to two successive puff actions detected by the puff detector 112a, time from the end time of a first puff action to the start time of a next, i.e., a second puff action, by using a timer.

As explained above, the detected-time corrector 106c corrects detected-time of puff action, according to the time correction model defined based on the atomization characteristic of the aerosol source with respect to the puff action. The detected-time accumulator 106d calculates accumulated detected-time by accumulating corrected detected-time of puff action. The inhaled-component-source remaining-quantity-level estimator 106e estimates the remaining quantity levels/level of the flavor source and/or the aerosol source, based on the accumulated detected-time. Further, it is judged that shortage in the remaining quantities (quantity) of the flavor source and/or the aerosol source has occurred, in the case that the lengths (length) of accumulated detected-time have (has) reached predetermined threshold lengths of time (a predetermined threshold length of time). The notification instructing unit 106f instructs the notifier 108 to perform notification operation, in response to a result of estimation of the remaining quantity levels/level of the flavor source and/or the aerosol source. Especially, in the case that it is judged in the inhaled-component-source remaining-quantity-level estimator 106e that shortage in the remaining quantity has occurred, the notifier 108 is operated in response thereto to output notification representing shortage in the remaining quantity.

Regarding the functional blocks in the sensor 112, the puff detector 112a detects puff action performed by a user and/or non-puff action, by using a suction sensor such as a microphone condenser, for example. The output unit 112b outputs various kinds of pieces of information detected by the sensor 112 to the controller 106, or stores them in the memory 114.

Regarding the information stored in the memory 114, the cartridge's maximum consumption time information 114a represents time information (for example 1000 seconds) corresponding to the maximum consumption quantities/quantity of the aerosol source and/or the flavor source held in the reservoir 116 of the cartridge. The capsule's maximum consumption time information 114b represents time information (for example 100 seconds) corresponding to the maximum consumption quantity of the flavor source 128 held in the capsule of the inhaler 100B. It is preferable that they be set, in advance, at the time of designing of the cartridge and the capsule, for example. Further, regarding the flavor source 128 held in the capsule, it is preferable that the values be set in such a manner that different values are set to correspond to respective kinds of flavor sources.

The time correction model information 114c is information relating to the above-explained atomization characteristic of the aerosol source and information relating to the time correction model based on the atomization characteristic of the aerosol source. For example, the time correction model information 114c comprises information representing 1.0 second and 2.4 seconds relating to the puff action periods shown in Fig. 3, information representing 10 seconds relating to the puff action interval shown in Fig. 4, information representing the set value "a" and the function "$y=C_1(x,a)$" of the time correction model shown in Fig. 5B, and information representing the value of the adjustment quantity "$T_0*(1-t_{int}/10)$" and the function "$y=C_2(x,t_{int})$" ($=C_1(x+T_0*(1-t_{int}/10),a)$) of the time correction model. The accumulated detected-time information 114d is information representing the accumulated detected-time accumulated by the detected-time accumulator 106d, and is updated each time when puff action is performed by a user.

(4) Process Flow for Controlling Operation of Electric Power Source Unit

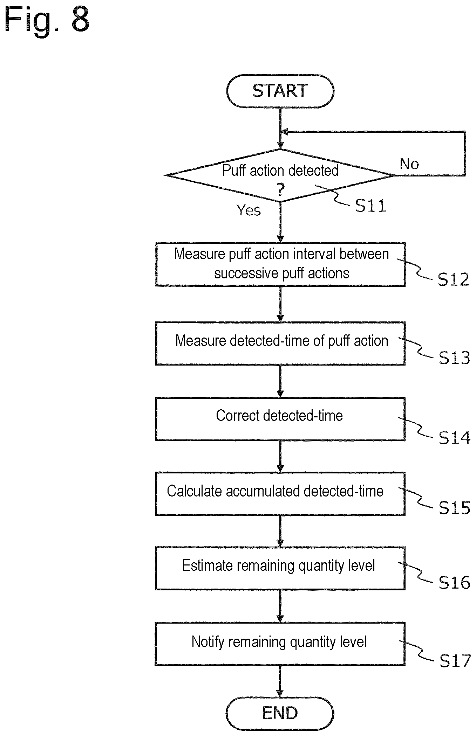

Each of Fig. 8 and Fig. 9 is an example of a process flow of control, performed by the controller 106, of operation of the electric power source unit 102 which is a component of the inhaler 100 according to the first embodiment. Fig. 8 is an example of an overall process flow of control, performed by the controller 106, of operation of the electric power source unit 102. Fig. 9 is an example of a detailed process flow relating to correction of detected-time of a puff action.

When the process flow in Fig. 8 is started, first, in step S11, the controller 106 makes the puff detector 112a in the sensor detect puff action performed by a user. Specifically, it is judged whether a puff action, which is defined by the start time and the end time of the puff action, is detected by the puff detector 112a. If puff action is detected (step S11: Yes), the puff-action-interval measuring unit 106b in the controller measures, in step S12, a puff action interval between two successive puff actions. Further, in step S13, the puff-detection-time measuring unit 106a in the controller measures the detected-time of the most recent puff action. The "detected-time" in this case is the time during that the detected puff action was continued. In this regard, the operation sequence of step S12 and step S13 may be reversed, and an optional process may be added between the above steps.

Next, in step S14, the detected-time corrector 106c in the controller corrects, based on the value of the characteristic parameter relating to the puff action, the detected-time of the puff action measured in step S13. Step 14 is based on the above-explained atomization characteristic of the aerosol source in the inhaler 100. Specifically, the time correction model 1 (Figs. 3, 5A, and 5B) based on the atomization characteristic 1 of the aerosol source is used, and the characteristic parameter in this case includes the detected-time of puff action. Similarly, the time correction model 2 (Figs. 4, 6A, and 6B) based on the atomization characteristic 2 of the aerosol source is also used, and the characteristic parameter in this case includes the puff action interval. Information of the time correction models 1 and 2 based on the atomization characteristics 1 and 2 is stored as a part of the time correction model information 114c in the memory 114 in advance.

Following the above, in step S15, the detected-time accumulator 106d in the controller calculates accumulated detected-time by accumulating the lengths of detected-time that have been corrected in step S14. The accumulated detected-time is stored, each time when it is updated, as a part of the accumulated detected-time information 114d in the memory 114. In next step S16, the inhaled-component-source remaining-quantity-level estimator 106e in the controller estimates, based on the accumulated detected-time calculated in step S15, the remaining quantity levels/level of the flavor source and/or the aerosol source. The remaining quantity level may be represented as that having any form, specifically, it may be calculated as puff time (in seconds) that is allowed to spend, or a percentage (%) of the puff time. Further, it is possible to perform judgment to judge that shortage in the remaining quantities (quantity) of the flavor source and/or the aerosol source has occurred, in the case that the accumulated lengths (length) of detected-time have (has) reached predetermined lengths of threshold time (a predetermined length of threshold time). The predetermined lengths (length) of threshold time are (is) stored in the memory 114 in advance as a part of capsule's maximum consumption time information 114b (for example, 100 seconds) and/or a part of the cartridge's maximum consumption time information 114a (for example, 1000 seconds).

Finally, in step S17, the notification instructing unit 106f in the controller instructs the notifier 108 to perform operation for notifying the remaining quantity levels/level estimated in step S16. For example, it is preferable to provide a user with notification in various forms, for example, lighting by an LED, displaying by a display, vocalization by a speaker, vibration by a vibrator, or the like, or a combination thereof. Especially, in the case that it is judged in step S16 that shortage in the remaining quantities/quantity of the flavor source and/or the aerosol source has occurred, it is preferable that the notifier 108 be operated to output notification representing shortage in the remaining quantities/quantity.

As explained above, in the first embodiment, the object of estimation of the remaining quantity level can be set flexibly, according to the structures of the inhalers 100A and 100B. Specifically, in the cases/case of the capsule 126 and/or the cartridge 104, processing required to be performed is, merely, converting the quantities/quantity of the inhaled component sources/source to time information, and storing the time information as the capsule's maximum consumption time information 114b and/or the cartridge's maximum consumption time information 114a. Since such time information only is used in the controller 106 when operation for estimating the remaining quantity level is performed, the operation is efficient.

With reference to Fig. 9, the process flow relating to correction of the detected-time of puff action in above-explained step S14 will be explained in detail. As explained above, the process in step S14 is performed by the detected-time corrector 106c in the controller. As shown in the figure, step S14 comprises processing operation comprising time correction 1 represented by steps S141 to S143 and time correction 2 represented by steps S144 to S146.

In the time correction 1, first, in step S141, it is judged whether the puff action interval tint between two successive puff actions, that was measured in step S12 in Fig. 8, is equal to or shorter than 10 seconds. The above judging process is associated with the atomization characteristic 2 of the aerosol source shown in Fig. 4, and also associated with the time correction model 2 that is based on the atomization characteristic 2 and shown in each of Figs. 6A and 6B.

That is, in the case that the puff action interval tint is equal to or shorter than 10 seconds (S141: Yes), the above-explained adjustment quantity "$T_0*(1-t_{int}/10)$" is calculated and added to the detected-time for adjustment, in step S142. Specifically, in the case that it is supposed that the corrected detected-time with respect to actual detected-time t of a puff action is $t_{crt1}$,

$$t_{crt1} = t + T_0 * (1 - t_{int} / 10)$$

is calculated. The above is calculated for performing, based on Formula 4, conversion

$$y = C_2 (x, t_{int}) = C_2 (t_{crt1}, 10) = C_1 (t_{crt1}, a)$$

by adjustment of time (Fig. 6B).

On the other hand, in the case that the puff action interval tint is longer than 10 seconds (S141: No), correction is not performed, and the adjustment quantity may merely be set to 0, in step S143. That is, it may be set as follows:

$$t_{crt1} = t$$

The reason that the adjustment quantity is set to 0 is that, according to the atomization characteristic 2 of the aerosol source, it can be regarded that negative correlation between puff action intervals and atomization quantities relating to puff actions does not occur in the case that the puff action interval is set to that equal to or longer than 10 seconds (the dotted line in Fig. 4), and, accordingly, it is not necessary to perform correction based on the time correction model 2.

Next, in the time correction 2, in step S144, it is judged whether the detected-time $t_{pf}$ of a puff action is equal to or shorter than 1 second. The above judging process is associated with the atomization characteristic 1 of the aerosol source shown in each of Figs. 3 and 5A, and also associated with the time correction model 1 that is based on the atomization characteristic 1 and shown in Fig. 5B.

That is, in the case that the detected-time $t_{pf}$ of the puff action is equal to or shorter than 1 second (S144: Yes), correction based on Formula 5, in the case when $0<x\leq1$, is performed. Specifically, in the case that it is supposed that the further corrected detected-time with respect to the corrected detected-time $t_{crt1}$ based on the time correction 1 is $t_{crt2}$,

$$t_{crt2} = a * t_{crt1}$$

may be calculated. In this regard, "a" is a constant that is set in advance, and $0<a<1$.

On the other hand, in the case that the puff action period $t_{pf}$ is longer than 1 second (S144: No), correction based on Formula 5, in the case when $1<x\leq2.4$, is performed. Specifically,

$$t_{crt2} = b * t_{crt1} - W_0$$
$$= ((2.4 - a) / 1.4) * t_{crt1} - (2.4 / 1.4) * (1 - a)$$

may be calculated.

As explained above, the calculation process of $t_{crt2}$ includes weighted calculation of the detected-time $t_{pf}$ using a multiplier (a or b) selected in relation to the detected-time $t_{pf}$ of the puff action. Specifically, the predetermined constant "a" is selected in the case that the detected-time $t_{pf}$ is equal to or shorter than 1.0 second, and, on the other hand, the constant "b" is selected in the case that the detected-time $t_{pf}$ is longer than 1.0 second. In this case, since $0<a<1$ and $b=(2.4-a)/1.4$, relationship $a<b$ is satisfied (Fig. 5B).

According to the first embodiment, the detected-time $t_{pf}$ of the puff action is appropriately corrected through the time correction models 1 and 2 shown in Fig. 9. That is, detected-time, that is more closely related to detected-time that is more closely related to an actual state, i.e., an actual consumption quantity of an aerosol source, and a quantity of aerosol that has actually passed through a flavor source (in other words, an actual flavor quantity given by the flavor source), can be calculated. As a result, accuracy at the time of estimation of the remaining quantity level can be improved.

In addition, according to the first embodiment, by dynamically grasping the remaining quantity level of the inhaled component source, operation of the inhaler 100 can be optimized. That is, the frequency of discarding of an inhaler, a battery, an inhaled article, or the like can be lowered by extending the span of life thereof, and an environmentally friendly inhaler can be provided by preventing unnecessary replacement of an inhaled component source. Thus, the first embodiment is advantageous in the point that it takes the perspective of energy conservation and environmental preservation into consideration.

In the above description, the operation method of the electric power source unit 102, which is a component of the inhaler, according to the first embodiment has been explained with reference to the block diagrams shown in Figs. 1A, 1B and 7, the graphs shown in Fig. 2 to Fig. 6B, and the processing flow shown in each of Figs. 8 and 9. It can be understood that the first embodiment can be implemented as a program which makes a processor, which is in the controller 106 in the electric power source unit 102, instruct the electric power source unit 102 to perform the processing flow shown in each of Figs. 8 and 9 when the program is executed by the processor. Similarly, it can be understood that it can be implemented as a computer-readable storage medium storing the above program.

< Second Embodiment >

A method for operating an electric power source unit in an inhaler according to a second embodiment of the present disclosure will be explained in the following description. In this regard, since the sections "(1) Basic Method for Estimating Remaining Quantity Level of Inhaled Component Source" and "(2-1) Atomization Characteristics of Aerosol Source" apply similarly to the second embodiment, explanation thereof will be omitted herein. Similar to the first embodiment, in the second embodiment, atomization characteristics 1 and 2 of the aerosol source explained in relation to Figs. 3 and 4 are further improved and the improved atomization characteristics are adopted. That is, in the second embodiment, a time correction model is also defined based on an atomization characteristic of an aerosol source in the inhaler 100.

(2-2) Time Correction Models Generated Based on Atomization Characteristics

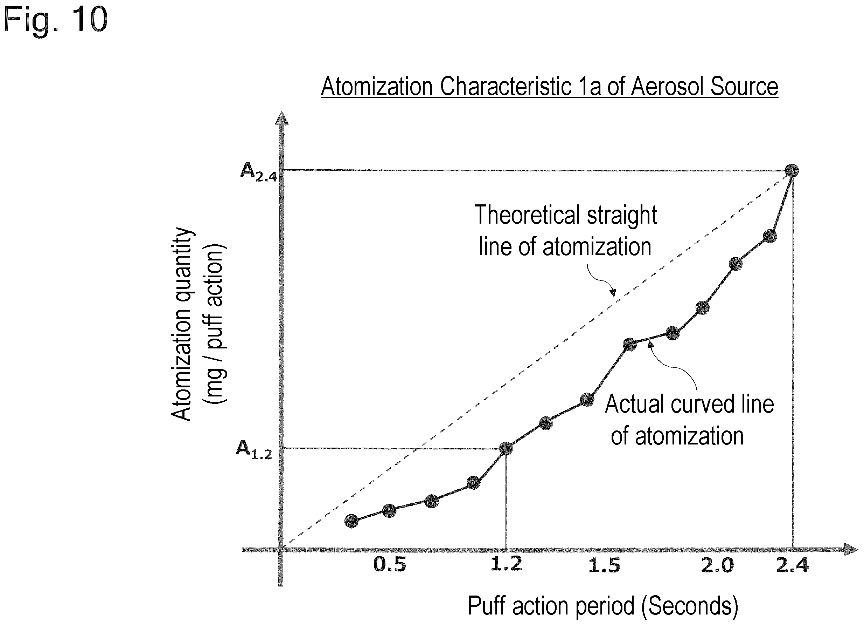

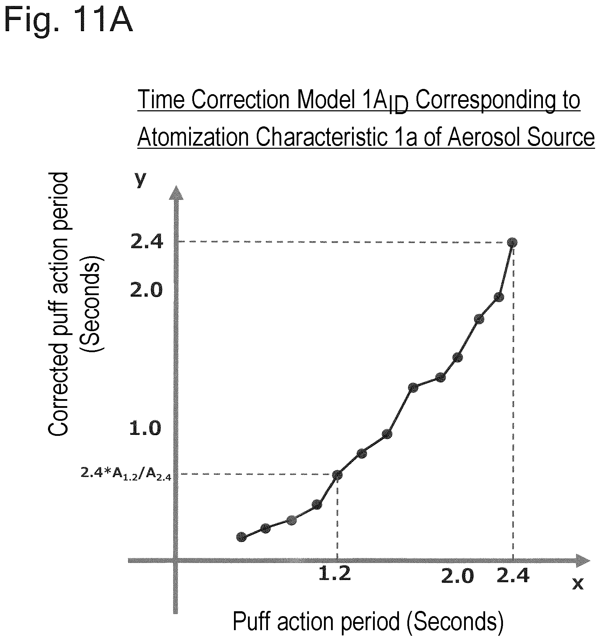

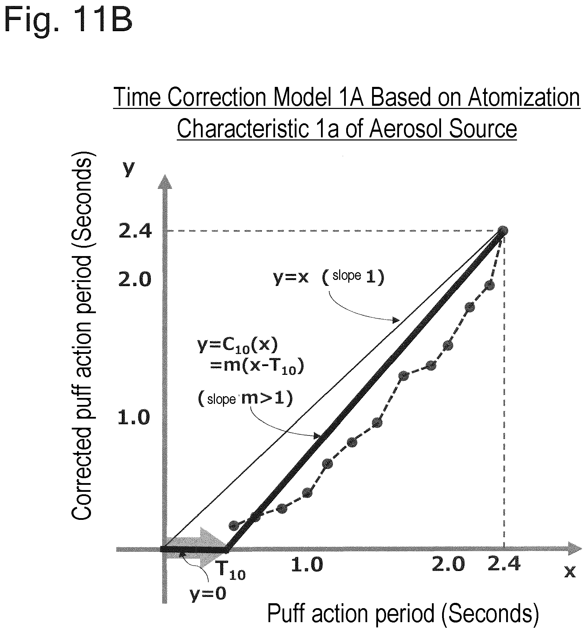

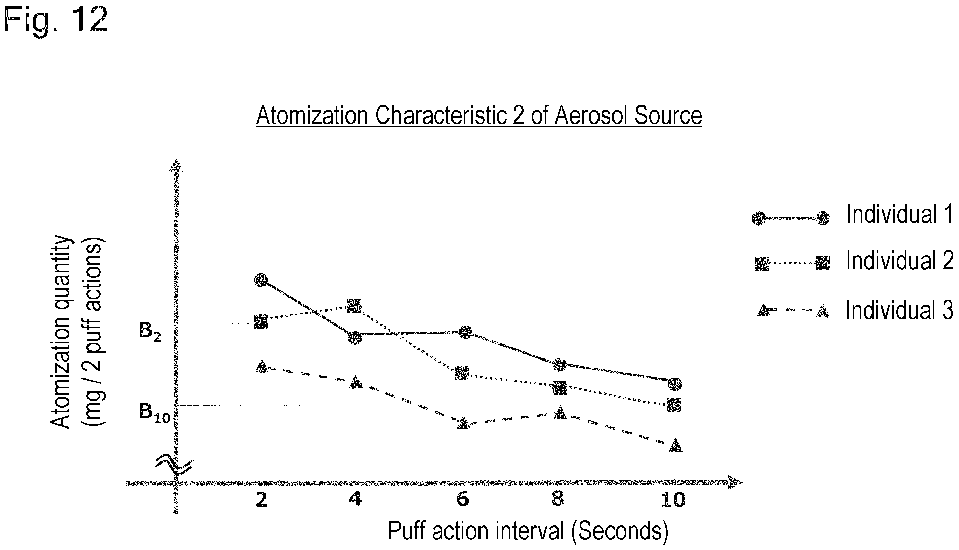

With reference to Fig. 10A to Fig. 13B, methods for generating time correction models 1A and 2A for correcting detected-time of detected puff actions, according to the atomization characteristics 1a and 2a of an aerosol source, will be explained. Fig. 10 and Fig. 12 are schematic figures for explaining the atomization characteristics 1a and 2a of the aerosol source. Each of Fig. 11A and Fig. 11B is a schematic figure for explaining the time correction model 1A based on the atomization characteristic 1a of the aerosol source, and each of Fig. 13A and Fig. 13B is a schematic figure for explaining the time correction model 2A based on the atomization characteristic 2a of the aerosol source.

[ Time Correction Model 1A Based on Atomization Characteristic 1a ]

Similar to the atomization characteristic 1 of the aerosol source shown in Fig. 3, Fig. 10 is a graph that shows an actual atomization line as a polygonal line graph, by using 13 sample points of the puff action periods and the atomization quantities of the aerosol source (and/or the flavor source), and defines the atomization characteristic 1a. The value of the atomization quantity at each sample point is that obtained by performing plural number of times of measurement of the atomization quantity of the aerosol source during each predetermined puff action period by performing an experiment, and calculating an average thereof.

As studied in relation to the above-explained atomization characteristic 1, an actual atomization quantity of an aerosol source is smaller than a theoretical atomization quantity. That is, in the case that an actual value of the puff action period, as it stand, is applied to estimation of the remaining quantity level to calculate an ideal value, the atomization quantity may be estimated as that larger than an actual atomization quantity, so that there may be a case that the quantity larger than the estimated quantity of the aerosol source may remain. That is, it is preferable that the value of the puff action period be used, after correcting it to be somewhat smaller, in estimation of the remaining quantity level. In this regard, similar to the first embodiment, the maximum value of the puff action period is set to 2.4 seconds according to the atomization characteristic 1 of the aerosol source (Fig. 3), in the second embodiment. Regarding 2.4 seconds, it is the value with respect to that the consumption efficiency of the aerosol source in the inhaler is the highest. However, the above value is a mere example, and it is preferable to set, in accordance with a device characteristic and/or a design of an inhaler, an ideal value with respect to that the consumption efficiency of the aerosol source in the inhaler is the highest.

Fig. 11A shows an example of a time correction model $1A_{ID}$ based on the atomization characteristic 1a of the aerosol source. The time correction model $1A_{ID}$ corresponds to the atomization characteristic 1a that is shown in Fig. 10 and obtained by performing an experiment. In the graph shown in Fig. 11A, the horizontal axis (x axis) represents a puff action period (in seconds) and the vertical axis (y axis) represents a corrected puff action period (in seconds) relating to the puff action period.

Specifically, it is preferable that the corrected puff action period be determined in accordance with a relative atomization quantity ratio with respect to each predetermined puff action period, by using, as the basis, 2.4 seconds that is an ideal value with respect to that the consumption efficiency of the aerosol source is the highest, in accordance with the atomization characteristic 1a in Fig. 10. For example, regarding the atomization characteristic 1a in Fig. 10, the atomization quantity when the puff action period is 2.4 seconds is set to $A_{2.4}$ mg, and the atomization quantity when the puff action period is 1.2 seconds is set to $A_{1.2}$ mg. In the above case, in Fig. 11A, it is preferable that the corrected puff action period (y) when the puff action period (y) is 1.2 seconds be calculated by $2.4*A_{1.2}/A_{2.4}$.

Fig. 11B shows an example of a time correction model 1A based on the atomization characteristic 1a. The time correction model 1A is theoretically defined in relation to an ideal time correction model $1A_{ID}$ in Fig. 11A. Similar to Fig. 11A, in the graph in Fig. 11B, the horizontal axis (x axis) represents a puff action period (in seconds) and the vertical axis (y axis) represents a corrected puff action period (in seconds) relating to the puff action period. That is, the time correction model 1A in Fig. 11B is a model based on puff action periods. Further, for maintaining the value of the corrected puff action period (y) to be 2.4 seconds when the puff action period (x) is 2.4 seconds, a function correlating to the time correction model $1A_{ID}$ in Fig. 11A is defined in the range $0<x\leq2.4$.

Specifically, as shown in Fig. 11B, a constant $T_{10}$, wherein y=0 holds when x=$T_{10}$, is adopted. Thus, the function of the time correction model 1A, y=$C_{10}(x)$, is represented by the following two linear functions (Formula 6) based on the puff action periods (x):

-    The case of $0 < x \leq T_{10}$

$$y = 0$$

-    The case of $T_{10} < x \leq 2.4$

$$y = m (x - T_{10})$$

(Provided that m>1)

In this regard, the slope m (>1) is determined by using the formula "$m=2.4/(2.4-T_{10})$" and set in the memory 114.

As explained above, by applying the time correction model 1A based on the puff action period, the value of the corrected puff action period (y) is made smaller than the value of the related puff action period (x), so that the value can be appropriately corrected to approach the ideal time correction model $1A_{ID}$ (the dotted line). In this regard, it is preferable that the constant $T_{10}$ be set to a value smaller than 1.0. Specifically, it is preferable that it be set in the memory 114, by taking a device characteristic of the inhaler 100 into consideration and obtaining it experimentally. The "device characteristic" in the present case may include a cartridge characteristic, a heating characteristic of a heater, and a loss characteristic relating to depositing of aerosol sources in a mouthpiece and/or a capsule; however, the characteristics included therein are not limited to the above listed characteristics.

In the present case, in the range near the point wherein the value of the puff action period (x) is $T_{10}$, the corrected puff action period (y) relating to the time correction model 1A is smaller than a corresponding value relating to the time correction model $1A_{ID}$ (the dotted line). However, according to an experiment performed by the inventors, it has been found that a puff action period having a value smaller than 1.0 second is rarely observed in puff action of a user and occurrence of such a case is rarely anticipated. That is, if $T_{10}$ is set to a value smaller than 1.0, it is not necessary to consider effect due to correction, originally (this will be explained later).

[ Time Correction Model 2A Based on Atomization Characteristic 2a ]

Similar to the atomization characteristic 2 of the aerosol source shown in Fig. 4, Fig. 12 is a graph that shows an actual atomization line by a polygonal line graph using 5 sample points of intervals and the atomization quantities of the aerosol source (and/or the flavor source), and defines the atomization characteristic 2a, wherein each interval is that between two successive puff actions. The value of the atomization quantity at each sample point is that obtained by performing an experiment, wherein measurement of the atomization quantity of the aerosol source with respect to each two-second puff action interval was performed. The puff action interval is measured by a sensor and a timer. In this regard, in Fig. 12, the puff action period was fixed to 2.4 seconds, and measurement was performed.

In this regard, the atomization quantity of the aerosol source relating to the puff action interval relates closely to a device characteristic, and there are large individual differences. Thus, in the example in Fig. 12, result of measurement using three individuals 1 to 3 is plotted. Further, similar to the first embodiment, in the second embodiment, the reference value of the puff action interval between two successive puff actions is set to 10 seconds, in accordance with the atomization characteristic 2 of the aerosol (Fig. 4). The above value, 10 seconds, is a value leading to the state wherein the consumed atomization quantity of the aerosol source, in relation to the puff action interval, is stabilized. However, the above value is a mere example, and it is preferable to set a preferred value determined by performing an experiment, in accordance with a device characteristic and/or a design of an inhaler.

As studied in relation to the above-explained atomization characteristic 2, negative correlation between puff action intervals and atomization quantities of the aerosol source (and/or the flavor source) occurs, in the case that the puff action interval is equal to or shorter than 10 seconds. That is, the atomization quantity of the aerosol source becomes smaller as the puff action interval becomes longer. Regarding the above matter, in the case that the puff action interval is equal to or shorter than 10 seconds, if an actual value of a puff action period is applied as it stands to estimation of a remaining quantity level, a remaining quantity smaller than an actual remaining quantity may be estimated, and, accordingly, shortage in the flavor source, that is contrary to the estimate, may occur. Thus, it is preferable to correct the value of the puff action period to make it somewhat larger, and use it in estimation of the remaining quantity level.

Fig. 13A shows an example of a time correction model $2A_{DIF}$ based on the atomization characteristic 2a of the aerosol source in Fig. 12. In the graph shown in Fig. 13A, the horizontal axis (v axis) represents an interval (in seconds) between two successive puff actions, and the vertical axis (w axis) represents a corrected difference puff action period (in seconds) relating to the puff action period. In this regard, for simplicity, in Fig. 13A, two data groups relating to the individuals 1 and 2 shown in relation to the atomization characteristic 2a in Fig. 12 only are shown (the dotted line and the broken line), and the data group relating to the individual 3 is omitted. In relation to the above data groups relating to the respective individuals, the time correction model $2A_{DIF}$ is defined (the solid line).

Specifically, it is preferable that the corrected difference puff action period (in seconds) of each individual, that is the sample point, be determined in accordance with a relative atomization quantity ratio with respect to each predetermined puff action interval, that is shown in Fig. 12, by using, as the basis, the matter that the value of the puff action interval is 10 seconds. For example, regarding the atomization characteristic 2a relating to the individual 2 in Fig. 12, the atomization quantity when the puff action period is 10 seconds is set to $B_{10}$ mg, and the atomization quantity when the puff action period is 2 seconds is set to $B_2$ mg. In the above case, in Fig. 13A, it is preferable that the corrected difference puff action period when the puff action interval is 2 seconds be calculated by $10*(B_2-B_{10})/B_{10}$. In this regard, it is preferable that the corrected difference puff action period be set to 0, in the case that the value of the puff action interval is larger than 10 seconds.

The time correction model $2A_{DIF}$ in Fig. 13A is that for calculating, as adjustment time, the corrected difference puff action period calculated based on the puff action interval, based on the interval between two successive puff actions. Further, as a result that the calculated adjustment time is added to the detected-time in the time correction model 1A based on the atomization characteristic 1a of the aerosol source, the time correction model 2A based on the atomization characteristic 2a is defined.

In more detail, it is preferable that the time correction model $2A_{DIF}$ based on the atomization characteristic 2a be defined as a linear function for classifying an area including all sample points (data groups) of plural individuals and an area other than the above area, in the vw plane (the first quadrant) in Fig. 13A. Specifically, the function $w=C_{20}(v)$ is set in such a manner that w=0 when v=10, and represented by the following two linear functions (Formula 7) based on the puff action intervals:

- The case of $0 < v \leq 10$

$$w = p\,(v - 10)$$

(Provided that p<0)
- The case of $10 < v$

$$w = 0$$

In this regard, the slope p (<0) is a constant that is determined in advance based on data groups of plural individuals and by using an arbitrary technique, and set in the memory 114.

By applying the time correction model $2A_{DIF}$ that is based on the atomization characteristic 2a as explained above, adjustment time, that is a corrected difference puff action period (w), relating to a value of a puff action interval (v) can be determined. Further, by combining the time correction model $2A_{DIF}$ with the above-explained time correction model 1A, a time correction model 2A based on the atomization characteristic 2a, that will be explained later, is defined. As a result, a value of adjustment time is added to a value of a corrected puff action period (y), so that the value of the corrected puff action period can be corrected appropriately based on a value of a puff action period.

Fig. 13B shows a time correction model 2A based on an atomization characteristic 2a such as that explained above. Similar to Fig. 11B, in a graph in Fig. 13B, the horizontal axis (x axis) represents a puff action period (in seconds) and the vertical axis (y axis) represents a corrected puff action period (in seconds) relating to the puff action period. In the present case, when the value of the puff action period (x) is 2.4 seconds, the value of the corrected difference puff action period (w), that is calculated based on the puff action interval (v) and in accordance with the time correction model $2A_{DIF}$, is added as the adjustment time b to the puff action period having the value of 2.4 seconds. In this manner, the function of the time correction model 2A for calculating the value of the corrected puff action period (y) is defined. In the present case, the puff action interval (v) is represented by $t_{int}$.

Specifically, the function $C_{30}(x, t_{int})$ of the time correction model 2A based on the atomization characteristic 2a is represented by the following two linear functions (Formula 8) based on the puff action periods:

- The case of $0 < x \leq T_{10}$

$$y = 0$$

- The case of $T_{10} < x \leq 2.4$

$$y = n\,(x - T_{10})$$

In this regard, since y=2.4+b when X=2.4, the slope n is represented by the following formula (Formula 9) based on Formula 7 and Formula 8:

$$n = (2.4 + b) / (2.4 - T_{10})$$
$$= (2.4 + p\,(t_{int} - 10)) / (2.4 - T_{10})$$

Thereafter, by substituting Formula 9 in Formula 8, the function $C_{30}(x, tint)$ of the time correction model 2A is represented by the following formulas (Formula 8'):

- The case of $0 < x \leq T_{10}$

$$y = 0$$

- The case of $T_{10} < x \leq 2.4$

$$y = ((2.4 + p\,(t_{int} - 10))\,/\,(2.4 - T_{10}))\,*\,(x - T_{10})$$

As explained above, "p" and "$T_{10}$" are constants that have been set in advance, thus, finally, the function $C_{30}(x, tint)$ of the time correction model 2A can be represented as a function of the puff action periods (x) and the puff action intervals $T_{int}$.

As explained above, the time correction models 1A and 2A are defined based on the atomization characteristics 1a and 2a of the aerosol source. By using the model, finally, as shown by Formula 8', the corrected puff action period (y) can be calculated from the puff action period (x) and the puff action intervals Tint, and the respective values of the constants p and $T_{10}$. That is, by measuring detected-time, that is a puff action period during that a detected puff action is continued, and a puff action interval between two successive puff actions, in response to detection, by a sensor 212, of puff action performed by a user by using the inhaler 100, and substituting the measured values in the puff action periods "x" and the puff action intervals "Tint" in Formula 8', the corrected puff action period can be obtained. In this regard, it is preferable that the constants p and $T_{10}$ be set appropriately in accordance with a device characteristic and/or the design of the inhaler 100, at the time of designing, for example.

(3) Functional Block Diagram Relating to Estimation of Remaining Quantity Level of Inhaled Component Source by Electric Power Source Unit

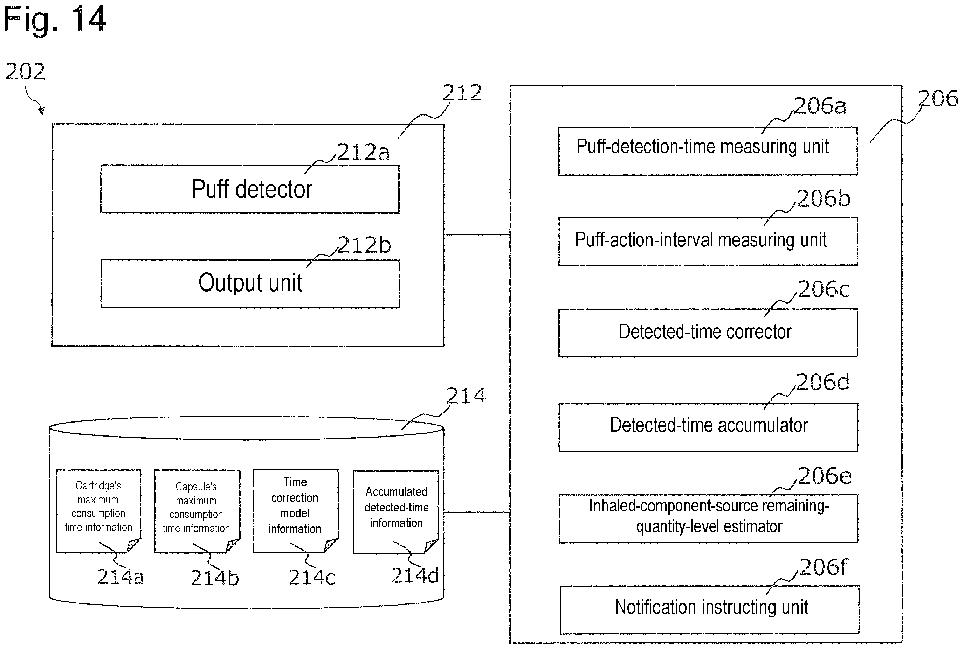

Fig. 14 relates to an electric power source unit 202 which is a component of an inhaler 100 according to the second embodiment, and shows examples of main functional blocks implemented by a controller 206 and a sensor 212, and examples of main pieces of information stored in a memory 214. Since the above components are similar to those in the first embodiment, outlines thereof only will be explained in the following description, and detailed explanation thereof will be omitted.

The controller 206 controls, in cooperation with the sensor 212 and the memory 214, various kinds of operation relating to estimation of the remaining quantity levels/level of the flavor source and/or the aerosol source. Examples of functional blocks of the controller 206 comprise a puff-detection-time measuring unit 206a, a puff-action-interval measuring unit 206b, a detected-time corrector 206c, a detected-time accumulator 206d, an inhaled-component-source remaining-quantity-level estimator 206e, and a notification instructing unit 206f. Examples of functional blocks of the sensor 212 comprise a puff detector 212a and an output unit 212b. An example of information stored in the memory 214 comprises time information such as cartridge's maximum consumption time information 214a, capsule's maximum consumption time information 214b, time correction model information 214c, and accumulated detected-time information 214d, and so on.

In the second embodiment, the puff-detection-time measuring unit 206a measures, with respect to puff action detected by the puff detector 212a, detected-time that is a puff action period during that a detected puff action is continued, and a puff action interval between two successive puff actions. The detected-time corrector 206c corrects detected-time of a puff action, by using a time correction model based on a characteristic parameter associated with puff action. In an example, the characteristic parameter comprises a puff action period and/or a puff action interval.

The detected-time accumulator 206d calculates accumulated detected-time by accumulating corrected detected-time of puff action. The inhaled-component-source remaining-quantity-level estimator 206e estimates the remaining quantity levels/level of the flavor source and/or the aerosol source, based on the accumulated detected-time. Further, it is judged that shortage in the remaining quantities (quantity) of the flavor source and/or the aerosol source has occurred, in the case that the accumulated lengths (length) of detected-time have (has) reached predetermined threshold lengths of time (a predetermined length of threshold time). The notification instructing unit 206f instructs the notifier 108 to perform notification operation, in response to a result of estimation of the remaining quantity levels/level of the flavor source and/or the aerosol source. Especially, in the case that it is judged in the inhaled-component-source remaining-quantity-level estimator 206e that shortage in the remaining quantity has occurred, the notifier 108 is operated in response thereto to output notification representing shortage in the remaining quantity.

(4) Process Flow for Controlling Operation of Electric Power Source Unit

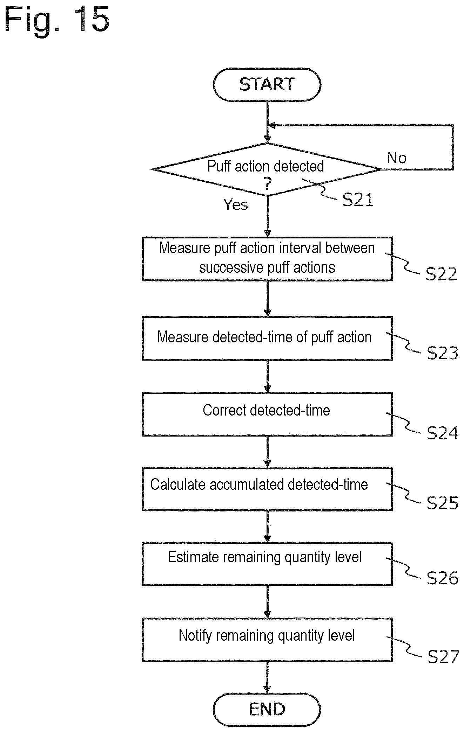

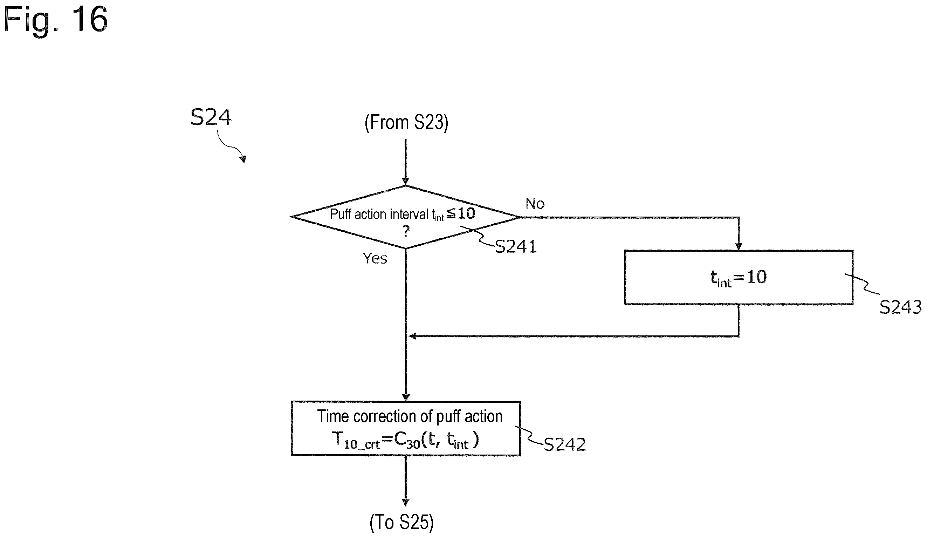

Each of Fig. 15 and Fig. 16 is an example of a process flow of control, performed by the controller 206, of operation of the electric power source unit 202 which is a component of the inhaler 100 according to the second embodiment. Fig. 15 is an example of an overall process flow of control, performed by the controller 206, of operation of the electric power source unit 202. Fig. 16 is an example of a detailed process flow relating to process S24 for correction of detected-time of a puff action. Regarding the flow in Fig. 15, since steps other than the step (S24) for correcting detected-time are similar to those in the first embodiment, outlines of them only will be explained in the following description, and detailed explanation thereof will be omitted.

When the process flow in Fig. 15 is started, first, in step S21, the controller 206 makes the puff detector 212a in the sensor detect puff action performed by a user. If a puff action is detected (step S21: Yes), the puff-action-interval measuring unit 206b in the controller measures, in step S22, a puff action interval between two successive puff actions. Further, in step S23, the puff-detection-time measuring unit 206a in the controller measures the detected-time of the most recent puff action. The "detected-time" is a puff action period during that the detected puff action is continued, and the value thereof will be corrected appropriately in a process after the present process.

Next, in step S24, the detected-time corrector 206c in the controller corrects, by using a time correction model based on a value of a characteristic parameter associated with puff action, the detected-time of the puff action measured in step S23. Specifically, the time correction models 1A and 2A are defined based on the atomization characteristics 1 (1a) and 2 (1a) of the aerosol source in the inhaler, and characteristic parameters include a puff action period and a puff action interval between two successive puff actions.

Following the above, in step S25, the detected-time accumulator 206d in the controller calculates accumulated detected-time by accumulating the lengths of detected-time that have been corrected in step S24. Next, in step S26, the inhaled-component-source remaining-quantity-level estimator 206e in the controller estimates, based on the accumulated detected-time calculated in step S25, the remaining quantity levels/level of the flavor source and/or the aerosol source. Further, it is possible to perform judgment to judge that shortage in the remaining quantities (quantity) of the flavor source and/or the aerosol source has occurred, in the case that the accumulated lengths (length) of detected-time have (has) reached predetermined threshold lengths of time (a predetermined threshold length of time). Finally, in step S27, the notification instructing unit 206f in the controller instructs the notifier 108 to perform operation for notifying the remaining quantity levels/level estimated in step S26. Especially, in the case that it is judged in step S26 that shortage in the remaining quantities/quantity of the flavor source and/or the aerosol source has occurred, it is preferable that the notifier 108 be operated to output notification representing shortage in the remaining quantities/quantity.

In the second embodiment, the object of estimation of the remaining quantity level can be set flexibly, according to the structures of the inhalers 100A and 100B. Specifically, in the cases/case of the capsule 126 and/or the cartridge 104, processing required to be performed is, merely, converting the quantities/quantity of the inhaled component sources/source to time information, and storing the time information as the capsule's maximum consumption time information 214b and/or the cartridge's maximum consumption time information 214a. Since such time information only is used in the controller 206 when operation for estimating the remaining quantity level is performed, the operation is efficient.

With reference to Fig. 16, the process flow relating to correction of the detected-time of puff action in above-explained step S24 will be explained in detail. As explained above, the process in step S24 is performed by the detected-time corrector 206c in the controller.

First, in step S241, it is judged whether the puff action interval tint between two successive puff actions, that was measured in step S22 in Fig. 15, is equal to or shorter than 10 seconds. The above judging process is associated with the atomization characteristics 2 and 2a of the aerosol source shown in Figs. 4 and 12, and also associated with the time correction models $2A_{DIF}$ and 2A that are based on the atomization characteristic 2a and shown in Figs. 13A and 13B.

It is supposed that corrected detected-time with respect to actual detected-time "t" of a puff action is $t_{10\_crt}$. In the case that the puff action interval tint is equal to or shorter than 10 seconds (S241: Yes), the corrected detected-time is calculated based on Formula 8' in step S242 as shown below;

$$t_{10\_crt} = C_{30}\,(t, t_{int})$$
$$= ((2.4 + p\,(t_{int} - 10)) / (2.4 - T_{10})) * (t - T_{10})$$

and outputted for next step S25 (Fig. 13B).

On the other hand, in the case that the puff action interval tint is longer than 10 seconds (S241: No), the puff action interval tint is set in such a manner that $t_{int}=10$ based on Formula 7, in step S243. Thereafter, the corrected detected-time is calculated based on Formula 8' in next step S242 as shown below;

$$t_{10\_crt} = C_{30}(t, 10)$$

$$= (2.4 / (2.4 - T_{10})) * (t - T_{10})$$

and outputted for next step S25 (Fig. 13B).

As explained above, according to the second embodiment, the value of the detected-time of the puff action is appropriately corrected through the time correction models 1A and 2A shown in Figs. 11B and 13B. That is, detected-time, that is more closely related to detected-time that is more closely related to an actual state, i.e., an actual consumption quantity of an aerosol source, and a quantity of aerosol that has actually passed through a flavor source (in other words, an actual flavor quantity given by the flavor source), can be calculated. As a result, accuracy at the time of estimation of the remaining quantity level can be improved.

(5) Modification Example of Second Embodiment

In the second embodiment, in the time correction model 2A that is based on the atomization characteristic 2a of the aerosol source, it is constructed in such a manner that the constant $T_{10}$ is adopted, and the corrected puff action period (y) becomes 0 in the case that the value of the puff action period (x) is equal to or smaller than $T_{10}$ (Formula 8 and Fig. 13B). The above construction is adopted based on result of study by the inventors, wherein the result is that, since there is a matter that a puff action such as that having a period smaller than 1.0 second is rarely observed and occurrence of such a puff action is rarely anticipated, it is not necessary to perform time correction with respect to a puff action period having a value smaller than 1.0 second, in view of the above matter. Tangible explanation thereof will be provided in the following description.

If it is supposed that a calculation process for time correction is to be performed with respect to a puff action having a period shorter than 1.0 second every time when such a puff action is observed, it is assumed that it does not meet the cost for calculation processing in the controller 206 in the electric power source unit 202. In more detail, for implementing a process for time correction such as that explained above, it is necessary to reserve a capacity in the memory 214 for storing an algorithm for the time correction. On the other hand, since occurrence of a puff action such as that having a period shorter than 1.0 second is rarely anticipated, it is assumed that such implementation is not balanced in the cost, since the cost for calculation processing thereof is too large. Further, originally, in a puff action such as that having a period shorter than 1.0 second, the consumed quantity of the inhaled component source is sufficiently small, so that it is assumed that it is not necessary to consider such a puff action.

Regarding the case when the value of $T_{10}$ is smaller than 1.0, the corrected puff action period (y) is set to 0 in the second embodiment, and, on the other hand, in the present modification example, the corrected puff action period (y) is not uniformly set to 0, and is set to a predetermined constant that is somewhat larger than 0. As a result, even in the case that puff actions, each having a period shorter than 1.0 second, only could be accepted due to occurrence of device failure, the value of the accumulated detected-time calculated by the detected-time accumulator 206d would be accumulated. That is, it is possible to use the value of the accumulated detected-time such as that explained above for detection of device failure, so that the span of life of the device can be extended.

Thus, in the modification example of the second embodiment, regarding a puff action period (detected-time) corrected by the time correction model 2B in the second embodiment, it is preferable that, if the value of the corrected detected-time is equal to or smaller than a predetermined constant, the value be uniformly updated to the value of the constant.

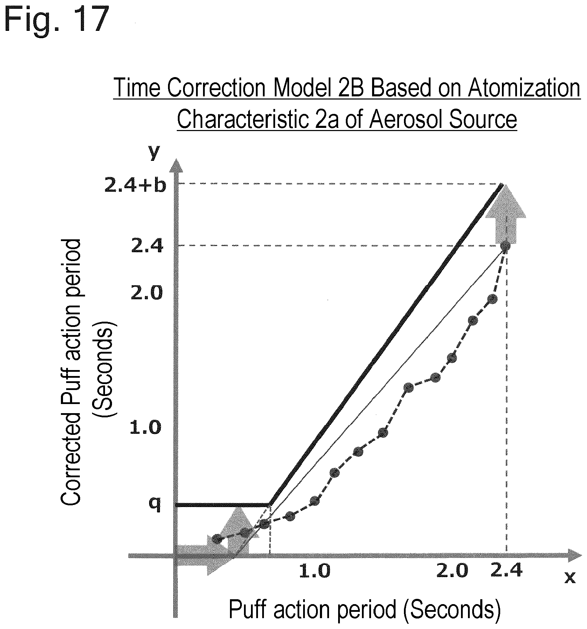

Fig. 17 shows a further example of a time correction model 2B based on the atomization characteristic 2a of the aerosol source, according to the modification example of the second embodiment. Similar to Fig. 13B, in the graph in Fig. 17, the horizontal axis (x axis) represents a puff action period (in seconds) and the vertical axis (y axis) represents a corrected puff action period (in seconds) relating to the puff action period. A function is further defined in relation to the time correction model 2B for uniformly updating the value of the corrected puff action period to q, in the case that the corrected puff action period is $0 < y \leq q$ in the time correction model 2A shown in Fig. 13B. In this regard, it is preferable that the constant q be obtained experimentally, wherein a device characteristic of the inhaler 100 may also be taken into consideration, and be set in the memory 214.

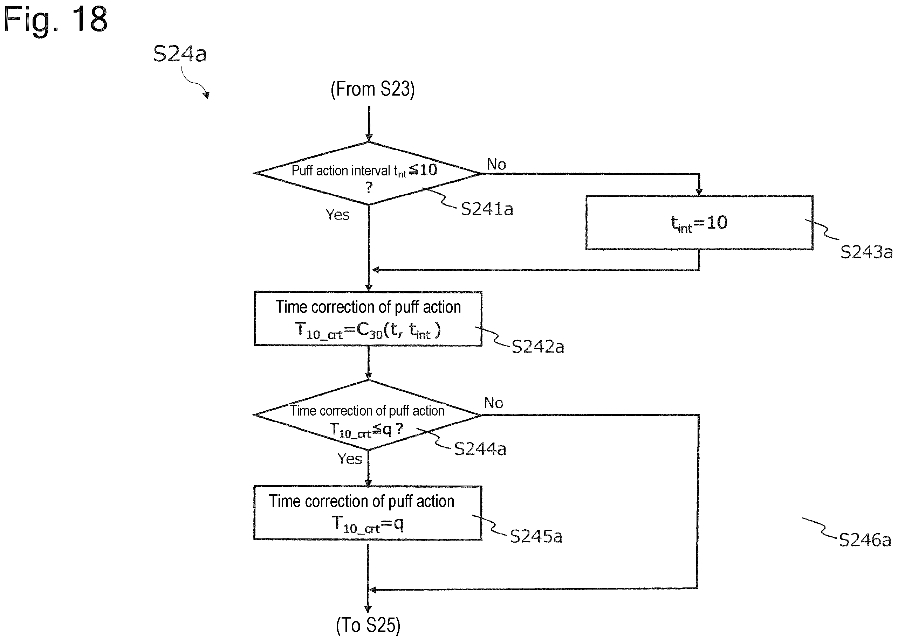

Fig. 18 is an example of a detailed process flow relating to process S24a for correcting detected-time of a puff action. Since steps S241a, S242a, and S243a are similar to steps S241, S242, and S243 in Fig. 16, explanation thereof will be omitted.

In the present case, regarding the output $t_{10\_crt} = C_{30}(t, t_{int})$ in step S242a, it is further judged whether the value of $t_{10\_crt}$ is equal to or smaller than q. In the case that the value of $t_{10\_crt}$ is equal to or smaller than q (S244a: Yes), the value of $t_{10\_crt}$ is updated to q, and outputted for next step S25. On the other hand, in the case that the value of $t_{10\_crt}$ is larger than q (S244a: No), the value of $t_{10\_crt}$, as it stands, is set as a corrected puff action period, and outputted for next step S25.

As explained above, according to the modification example of the second embodiment, in the case that a value of a calculated corrected puff action period is equal to or smaller than a predetermined constant, the value is uniformly updated to q, so that the calculation processing load on the controller 206 in the electric power source unit 202 can be reduced, and, further, device failure can be detected.

In the above description, the operation method of the electric power source unit 202, which is a component of the inhaler, according to each of the second embodiment and the modification example thereof has been explained with reference to the block diagrams shown in Figs. 1A, 1B and 14, the graphs shown in Figs. 10-13B and 17, and the processing flow shown in each of Figs. 15, 16 and 18. It can be understood that the second embodiment can be implemented as a program which makes a processor, which is in the controller 206 in the electric power source unit 202, instruct the electric power source unit 202 to perform the processing flow shown in each of Figs. 15, 16, and 18 when the program is executed by the processor. Similarly, it can be understood that it can be implemented as a computer-readable storage medium storing the above program.

< Third Embodiment >

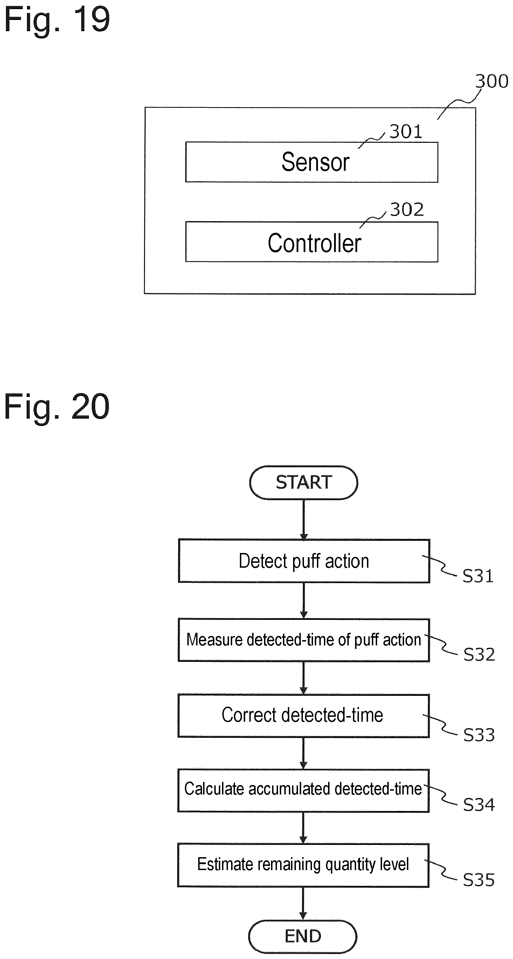

A method for operating an electric power source unit in an inhaler according to a third embodiment of the present disclosure will be explained in the following description. Fig. 19 is a block diagram showing a construction example of an electric power source unit 300 for the inhaler 100 in the third embodiment. In the example shown in Fig. 19, the electric power source unit 300 comprises a sensor 301 and a controller 302.

The sensor 301 corresponds to the sensor 112 shown in each of Figs. 1A and 1B and the sensor 212 shown in Fig. 14, for example. Also, the controller 302 corresponds to the controller 106 shown in each of Figs. 1A and 1B and the controller 206 shown in Fig. 14, for example.

The sensor 301, which is a component of the electric power source unit 300, detects puff action performed by a user. The controller 302, which is a component of the electric power source unit 300, measures detected-time that is a puff action period during that a puff action detected by the sensor 301 is continued. Further, the controller 302 corrects detected-time by using each time correction model that is based on an atomization characteristic of an aerosol source in puff action, and accumulates the corrected lengths of detected-time to calculate accumulated detected-time. Further, the controller 302 estimates, based on the accumulated lengths (length) of detected-time, remaining quantity levels (a remaining quantity level) of a flavor source and/or an aerosol source.

Fig. 20 is an example of a process flow of control, performed by the controller 302, of operation of the electric power source unit 300 which is a component of the inhaler 100, according to the third embodiment. When the process flow in Fig. 20 is started, first, in step S31, the controller 302 makes the sensor 301 detect puff action performed by a user. Next, in step S32, the controller 302 measures detected-time that is a puff action period during that the puff action detected by the sensor 301 is continued. Next, in step S33, the controller 302 corrects the detected-time by using each time correction model that is based on an atomization characteristic of an aerosol source in puff action. Next, in step S34, the controller 302 accumulates the detected-time corrected in S33 to calculate accumulated detected-time. Further, in step S35, the controller 302 estimates, based on the accumulated lengths (length) of detected-time calculated in S34, remaining quantity levels (a remaining quantity level) of the flavor source and/or the aerosol source.

According to the third embodiment, the controller 302 corrects, based on an atomization characteristic of an aerosol source, that is connected to a puff action detected by the sensor 301, detected-time of the puff action detected by the sensor and accumulates the corrected detected-time to calculates accumulated detected-time. Specifically, for example, the controller 302 corrects detected-time to make it large or small, based on a puff action period and a puff action interval (for example, an interval between an end of a puff action and a start of a next puff action) detected by the sensor 301, and accumulates it to calculate accumulated detected-time. Finally, the controller 302 estimates, based on the calculated accumulated lengths (length) of detected-time, remaining quantity levels (a remaining quantity level) of the flavor source and/or the aerosol source. As a result, appropriate grasping and notifying of remaining quantity levels (a remaining quantity level) of a flavor source and/or an aerosol source can be realized.

Thus, effect similar to that achieved by the first embodiment and/or the second embodiment, that have been explained above, can be achieved by the third embodiment.

Further, according to the third embodiment, by dynamically grasping the remaining quantity level of the inhaled component source, operation of the inhaler 100 can be optimized. That is, the frequency of discarding of an inhaler, a battery, an inhaled article, or the like can be lowered by extending the span of life thereof, and an environmentally friendly inhaler can be provided by preventing unnecessary replacement of an inhaled component source. Thus, the embodiment is advantageous in the point that it takes the perspective of energy conservation and environmental preservation into consideration.

In the above description, the operation method of the electric power source unit 300, which is a component of the inhaler, according to the third embodiment has been explained with reference to the block diagram shown in Fig. 19 and the processing flow shown in Fig. 20. It can be understood by a person skilled in the art that the third embodiment can be implemented as a program which makes a processor, which is in the controller 302 in the electric power source unit 300, instruct the electric power source unit 300 to perform the processing flow shown in Fig. 20 when the program is executed by the processor. Similarly, it can be understood by a person skilled in the art that it can be implemented as a computer-readable storage medium storing the above program.

In the above description, embodiments of the present disclosure have been explained; and, in this regard, it should be understood that they are mere examples, and they are not those limiting the scope of the present disclosure. It should be understood that change, addition, and modification with respect to the embodiments can be performed appropriately, without departing from the gist and the scope of the present disclosure. The scope of the present disclosure should not be limited by any of the above-explained embodiments, and should be defined by the claims and equivalents thereof only.

REFERENCE SIGNS LIST

100A, 100B, 100 ... Inhaler: 102 ... First member (Electric power source unit): 104 ... Second member (Cartridge): 106, 206 ... Controller: 108 ... Notifier: 110 ... Battery: 112, 212 ... Sensor: 114,214 ... Memory: 116 ... Reservoir: 118 ... Atomizer: 120 ... Air taking-in flow path: 121 ... Aerosol flow path: 122 ... Suction opening part: 126 ... Third member (Capsule): 128 ... Flavor source: 106a, 206a ... Puff-detection-time measuring unit: 106b, 206b ... Puff-action-interval measuring unit: 106c, 206c ... Detected-time corrector; 106d, 206d ... Detected-time accumulator: 106e, 206e ... Inhaled-component-source remaining-quantity-level estimator: 106f, 206f ... Notification instructing unit: 112a, 212a ... Puff detector: 112b, 212b ... Output unit: 114a, 214a ... Cartridge's maximum consumption time information: 114b, 214b ... Capsule's maximum consumption time information: 114c, 214c ... Time correction model information: 114d, 214d ... Accumulated detected-time information: 300 ... Electric power source unit: 301 ... Sensor: 302 ... Controller

**Claims**

1.  A method for operating an electric power source unit for an inhaler, comprising:

    making a sensor detect a puff action performed by a user;
    measuring detected-time that is a puff action period during that the detected puff action is continued;
    correcting the measured detected-time, by using a time correction model that is based on a characteristic parameter associated with the puff action;
    calculating accumulated detected-time by accumulating the corrected detected-time; and
    estimating a remaining quantity level of an inhaled component source, based on the accumulated detected-time.

2.  The method according to Claim 1, wherein the time correction model is defined based on an atomization characteristic of the inhaled component source in the inhaler.

3.  The method according to Claim 1 or 2, wherein the characteristic parameter comprises the puff action period.

4.  The method according to Claim 3, wherein
    the time correction model based on the puff action period is defined to include maintaining the corrected detected-time to be the first time, when a value of the measured detected-time is first time.

5.  The method according to Claim 4, wherein
    the time correction model based on the puff action period is defined to include reducing the measured detected-time in accordance with a first function of the detected-time, when the value of the measured detected-time is smaller than the first time.

6.  The method according to Claim 4 or 5, wherein the first time is 2.4 seconds.

7.  The method according to any one of Claims 1-6 further comprising: measuring a puff action interval between two successive puff actions; wherein
    the characteristic parameter comprises the puff action interval.

8.  The method according to Claim 7, wherein
    the time correction model based on the puff action interval is defined to include adding adjustment time, that is calculated based on the puff action interval, to the detected-time.

**9.** The method according to Claim 8, wherein
the time correction model based on the puff action interval is defined to include setting the adjustment time to 0, when a value of the puff action interval is larger than second time.

**10.** The method according to Claim 9, wherein
the time correction model based on the puff action interval is defined to include calculating the adjustment time in accordance with a second function of the puff action interval, when the value of the puff action interval is equal to or smaller than the second time.

**11.** The method according to Claim 9 or 10, wherein the second time is 10 seconds.

**12.** The method according to any one of Claims 1-11, wherein
said correcting the detected-time further updates, when a value of the detected-time corrected based on a value of the characteristic parameter is equal to or smaller than predetermined third time, the value of the corrected detected-time to the third time.

**13.** The method according to any one of Claims 1-12, wherein
said estimating comprises judging that shortage in the remaining quantity of the inhaled component source has occurred, when the accumulated detected-time has reached predetermined fourth time.

**14.** The method according to Claim 13 further comprising: making a notifier, which is a component of the electric power source unit, operate to provide notification representing shortage in the remaining quantity, in response to the judgement of occurrence of shortage in the remaining quantity of the inhaled component source.

**15.** A computer readable medium storing a computer-executable instruction, wherein a processor in the electric power source unit is made to perform the method according to any one of Claims 1-14 when the computer-executable instruction is executed.

**16.** An electric power source unit, which comprises a sensor for detecting a puff action performed by a user and a controller, for an inhaler: wherein the controller performs:

measurement of detected-time that is a puff action period during that the detected puff action is continued;
correction of the detected-time, by using a time correction model that is based on an atomization characteristic of an inhaled component source in the puff action;
calculation of accumulated detected-time by accumulating the corrected detected-time; and
estimation of a remaining quantity level of the inhaled component source, based on the accumulated detected-time.

**17.** The electric power source unit according to Claim 16, wherein
the estimation of the remaining quantity level includes judging that shortage in the remaining quantity of the inhaled component source has occurred, when the accumulated detected-time has reached predetermined threshold time.

**18.** The electric power source unit according to Claim 17 further comprising a notifier, wherein
the controller makes the notifier operate to provide notification representing shortage in the remaining quantity, in response to the judgement of occurrence of shortage in the remaining quantity.

**19.** The electric power source unit according to any one of Claims 16-18, wherein
the time correction model is defined as a function of the puff action period and a puff action interval between two successive puff actions.

# Fig. 1A

100A

# Fig. 1B

100B

Fig. 2

# Fig. 3

Atomization Characteristic 1 of Aerosol Source

Theoretical straight line of atomization

(Difference 2)

(Difference 1)

Actual curved line of atomization

Atomization quantity (mg / puff action)

Puff action period (Seconds)

EP 3 995 019 A1

# Fig. 4

Atomization Characteristic 2 of Aerosol Source

Fig. 5A

Atomization Characteristic 1 of Aerosol Source

# Fig. 5B

Time Correction Model 1 Based on
Atomization Characteristic 1 of Aerosol Source

$y=C_1(x, 0.7)$

Slope b
$(b=(2.4-a)/1.4)$

$y=C_1(x, a)$
(Provided that $0<a\leq1$)

Slope a

Corrected puff action period (Seconds)

Puff action period (Seconds)

# Fig. 6A

Time Correction Model 2 Based on
Atomization Characteristic 2 of Aerosol Source

# Fig. 6B

Time Correction Model 2 Based on
Atomization Characteristic 2 of Aerosol Source

## Fig. 7

**102**

**112**

**112a** Puff detector

**112b** Output unit

**114**

| 114a | 114b | 114c | 114d |
|---|---|---|---|
| Cartridge's maximum consumption time information | Capsule's maximum consumption time information | Time correction model information | Accumulated detected-time information |

**106**

**106a** Puff-detection-time measuring unit

**106b** Puff-action-interval measuring unit

**106c** Detected-time corrector

**106d** Detected-time accumulator

**106e** Inhaled-component-source remaining-quantity-level estimator

**106f** Notification instructing unit

EP 3 995 019 A1

# Fig. 8

START

Puff action detected ? —— No

S11

Yes

Measure puff action interval between successive puff actions
S12

Measure detected-time of puff action
S13

Correct detected-time
S14

Calculate accumulated detected-time
S15

Estimate remaining quantity level
S16

Notify remaining quantity level
S17

END

# Fig. 9

S14

(From S13)

Puff action interval $t_{int} \leqq 10$ ? —No→    S141

Yes

**Time correction 1**

Time correction 1 of puff action
$$t_{crt1}=t+T_0*(1-t_{int}/10)$$   S142

(No time correction)
$$t_{crt1}=t$$   S143

Puff action detected-time $T_{pf} \leqq 1$ ? —No→   S144

Yes

**Time correction 2**

Time correction 2a of puff action
$$T_{crt2}=a*T_{crt1}$$   S145

Time correction 2b of puff action
$$T_{crt2}=b*T_{crt1}-W0$$   S146

(To S15)

Fig. 10

Atomization Characteristic 1a of Aerosol Source

Theoretical straight line of atomization

Actual curved line of atomization

Puff action period (Seconds)

Atomization quantity (mg / puff action)

$A_{2.4}$

$A_{1.2}$

# Fig. 11A

Time Correction Model 1A$_{\text{ID}}$ Corresponding to
Atomization Characteristic 1a of Aerosol Source

# Fig. 11B

Time Correction Model 1A Based on Atomization Characteristic 1a of Aerosol Source

y — Corrected puff action period (Seconds)

x — Puff action period (Seconds)

$y=x$ (slope 1)

$y=C_{10}(x)$
$=m(x-T_{10})$

(slope $m>1$)

$T_{10}$

$y=0$

2.4, 2.0, 1.0 (y-axis); 1.0, 2.0, 2.4 (x-axis)

# Fig. 12

Atomization Characteristic 2 of Aerosol Source

EP 3 995 019 A1

# Fig. 13A

## Time Correction Model 2A$_{DIF}$ Based on Atomization Characteristic 2a of Aerosol Source

w

Corrected difference puff action period (Seconds)

$w = C_{20}(v)$
$= p(v - 10)$
$(p < 0)$

Individual 1
Individual 2

$w = 0$

2    4    6    8    10    v

Puff action interval (Seconds)

# Fig. 13B

Time Correction Model 2A Based on Atomization Characteristic 2a of Aerosol Source

# Fig. 14

# Fig. 15

```
                    ┌──────────┐
                    │  START   │
                    └────┬─────┘
                         │        ┌──────────────────┐
                         ▼        │                  │
                  ╱────────────╲  No                 │
             ╱ Puff action detected ╲────────────────┘
             ╲        ?         ╱  S21
                  ╲────┬───────╱
                    Yes │
                        ▼
        ┌─────────────────────────────────┐
        │ Measure puff action interval between │  S22
        │      successive puff actions     │
        └────────────────┬────────────────┘
                         ▼
        ┌─────────────────────────────────┐
        │  Measure detected-time of puff action │  S23
        └────────────────┬────────────────┘
                         ▼
        ┌─────────────────────────────────┐
        │       Correct detected-time      │  S24
        └────────────────┬────────────────┘
                         ▼
        ┌─────────────────────────────────┐
        │ Calculate accumulated detected-time │  S25
        └────────────────┬────────────────┘
                         ▼
        ┌─────────────────────────────────┐
        │    Estimate remaining quantity level │  S26
        └────────────────┬────────────────┘
                         ▼
        ┌─────────────────────────────────┐
        │   Notify remaining quantity level │  S27
        └────────────────┬────────────────┘
                         ▼
                    ┌──────────┐
                    │   END    │
                    └──────────┘
```

# Fig. 16

S24

(From S23)

Puff action interval $t_{int} \leqq 10$ ? — S241

No → $t_{int}=10$ — S243

Yes ↓

Time correction of puff action
$T_{10\_crt}=C_{30}(t,\ t_{int})$ — S242

(To S25)

EP 3 995 019 A1

Fig. 17

Time Correction Model 2B Based on Atomization
Characteristic 2a of Aerosol Source

## Fig. 18

S24a

(From S23)

Puff action interval $t_{int} \leqq 10$ ? — No → $t_{int}=10$ — S243a

S241a

Yes

Time correction of puff action
$T_{10\_crt}=C_{30}(t, t_{int})$ — S242a

Time correction of puff action
$T_{10\_crt} \leqq q$ ? — No — S244a

S244a

Yes

Time correction of puff action
$T_{10\_crt}=q$ — S245a

S246a

(To S25)

EP 3 995 019 A1

# Fig. 19

300

301

Sensor

302

Controller

# Fig. 20

START

Detect puff action — S31

Measure detected-time of puff action — S32

Correct detected-time — S33

Calculate accumulated detected-time — S34

Estimate remaining quantity level — S35

END

## INTERNATIONAL SEARCH REPORT

| | International application No. |
|---|---|
| | PCT/JP2020/025857 |

A. CLASSIFICATION OF SUBJECT MATTER
Int.Cl. A24F47/00(2020.01)i, A24F40/50(2020.01)i
FI: A24F40/50, A24F47/00

According to International Patent Classification (IPC) or to both national classification and IPC

B. FIELDS SEARCHED

Minimum documentation searched (classification system followed by classification symbols)
Int.Cl. A24F40/50, A24F47/00

Documentation searched other than minimum documentation to the extent that such documents are included in the fields searched
Published examined utility model applications of Japan      1922–1996
Published unexamined utility model applications of Japan    1971–2020
Registered utility model specifications of Japan            1996–2020
Published registered utility model applications of Japan    1994–2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y A | JP 2017-192393 A (JAPAN TOBACCO INC.) 26.10.2017 (2017-10-26), paragraphs [0060]-[0098], [0110]-[0134], [0172], [0173] | 1-7, 12-19 8-11 |
| Y | WO 2018/224823 A1 (NICOVENTURES HOLDINGS LIMITED) 13.12.2018 (2018-12-13), page 5, line 13 to page 6, line 19, page 10, lines 13-21 | 1-7, 12-19 |
| Y | KR 10-2018-0070443 A (KT&G CORPORATION) 26.06.2018 (2018-06-26), paragraphs [0042]-[0052] | 7, 12-15, 19 |
| A | JP 2017-532011 A (NICOVENTURES HOLDINGS LTD.) 02.11.2017 (2017-11-02), entire text, all drawings | 1-19 |
| A | WO 2017/057286 A1 (JAPAN TOBACCO INC.) 06.04.2017 (2017-04-06), entire text, all drawings | 1-19 |

☐ Further documents are listed in the continuation of Box C.          ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10.09.2020 | 24.09.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

| International application No. |
| --- |
| PCT/JP2020/025857 |

| | | |
| --- | --- | --- |
| JP 2017-192393 A | 26.10.2017 | US 2017/0042251 A1<br>paragraphs [0075]-[0116],<br>[0129]-[0156], [0200]<br>WO 2015/166952 A1<br>EP 3138424 A1<br>CN 106231936 A<br>KR 10-2016-0137627 A |
| WO 2018/224823 A1 | 13.12.2018 | JP 2020-522272 A<br>paragraphs [0019]-[0021], [0037]<br>US 2020/0163387 A1<br>EP 3634161 A1<br>KR 10-2019-0140477 A<br>CN 110719739 A |
| KR 10-2018-0070443 A | 26.06.2018 | JP 2020-516262 A<br>paragraphs [0043]-[0053]<br>US 2020/0086068 A1<br>WO 2018/190586 A2 |
| JP 2017-532011 A | 02.11.2017 | US 2017/0251725 A1<br>entire text, all drawings<br>WO 2016/030661 A1<br>EP 3185941 A1<br>CN 106604654 A<br>KR 10-2017-0041772 A |
| WO 2017/057286 A1 | 06.04.2017 | US 2018/0220711 A1<br>entire text, all drawings<br>WO 2017/056282 A1<br>EP 3348154 A1<br>KR 10-2018-0044409 A<br>CN 108135271 A |

Form PCT/ISA/210 (patent family annex) (January 2015)

**REFERENCES CITED IN THE DESCRIPTION**

**Patent documents cited in the description**

- JP 2014501105 A
- JP 2015531600 A
- JP 2017538410 A
- JP 2016525367 A
- JP 2019500896 A
- JP 2014501107 A